# EUROPEAN PATENT SPECIFICATION

(11) **EP 2 527 351 B1**
(45) Date of publication and mention of the grant of the patent: **11.12.2013**
(21) Application number: 12169491.3
(22) Date of filing: 25.05.2012
(51) Int. Cl.: C07F 9/6558, C07F 9/6561, A61K 31/675, A61P 31/04

(54) **Lipophosphonoxins, method of their preparation and use**
Lipophosphonoxine, Verfahren zur Herstellung und Verwendung
Lipophosphonoxines, leur procédé de préparation et d'utilisation

(30) Priority: 26.05.2011 CZ 20110312
(43) Date of publication of application: 28.11.2012
(73) Proprietor: Ustav organicke chemie a biochemie AV CR, v.v.i., 166 10 Praha 6 (CZ); Mikrobiologicky ustav AV CR, v.v.i., 142 20 Praha 4 - Krc (CZ); Ustav molekularni genetiky AV CR, v.v.i., 142 20 Praha 4 (CZ); Trios, spol. s r.o., 14900 Praha 4 (CZ)
(72) Inventor: Rejman, Dominik, 17000 Praha 7 (CZ); Pohl, Radek, 25264 Uholicky (CZ); Bartunek, Petr, 11000 Praha 1 (CZ); Ribeiro Pombinho, Antonio Jose, Golfeiras, Mirandela (PT); Krasny, Libor, 25263 Roztoky u Prahy (CZ); Latal, Tomas, 78314 Hlusovice (CZ)
(74) Representative: Gabrielova, Marta

(56) References cited:
- SUK ET AL: "Phosphonoxins: Rational design and discovery of a potent nucleotide anti-Giardia agent", BIOORGANIC & MEDICINAL CHEMISTRY LETTERS, PERGAMON, ELSEVIER SCIENCE, GB, vol. 17, no. 10, 27 April 2007 (2007-04-27), pages 2811-2816, XP022049593, ISSN: 0960-894X, DOI: 10.1016/J.BMCL.2007.02.063
- DOMINIK REJMAN ET AL: "Lipophosphonoxins: New Modular Molecular Structures with Significant Antibacterial Properties", JOURNAL OF MEDICINAL CHEMISTRY, vol. 54, no. 22, 24 November 2011 (2011-11-24), pages 7884-7898, XP55036669, ISSN: 0022-2623, DOI: 10.1021/jm2009343

## Description

### Field of the Invention

The invention relates to novel compounds with antibacterial properties, methods of synthesis thereof, and utilisation thereof *in vitro* and *in vivo.*

### Background Art

The advantages offered by antibiotics in the treatment of infectious diseases are endangered due to the increase in the number of antibiotic-resistant bacterial strains. This reduces the efficiency of antibiotic treatments and poses a serious health and economical problem. Currently, the need for novel antibiotics is becoming increasingly apparent (Davies D., Davies J., Microbiol. Mol. Biol. Rev. 2010, 74(3), 417; Kesselheim A. S., Outterson K., Health Aff. 2010, 29, 1689).

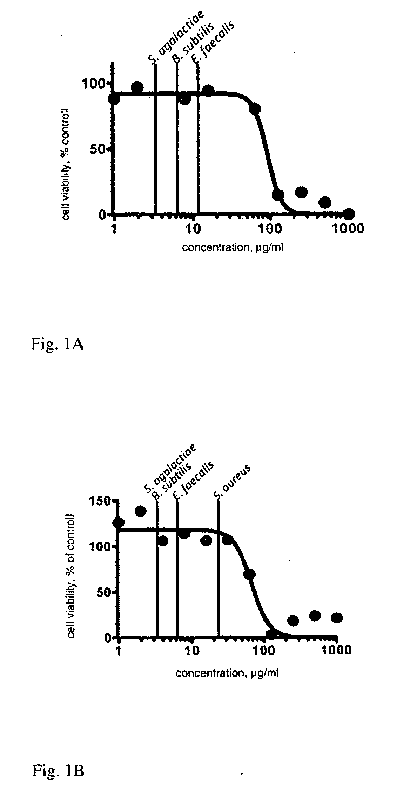

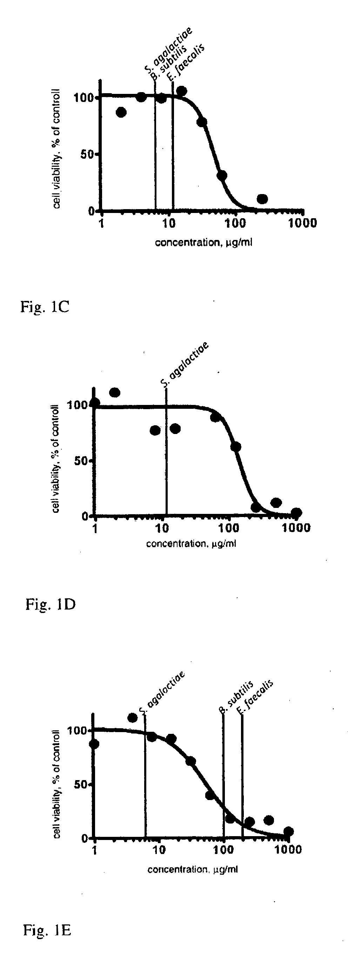

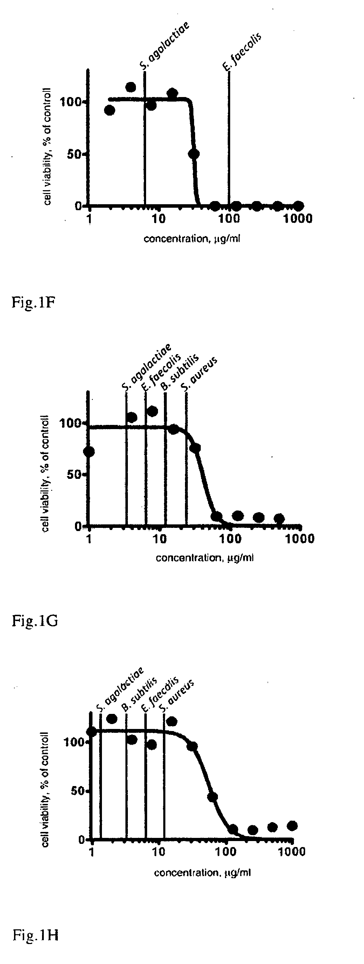

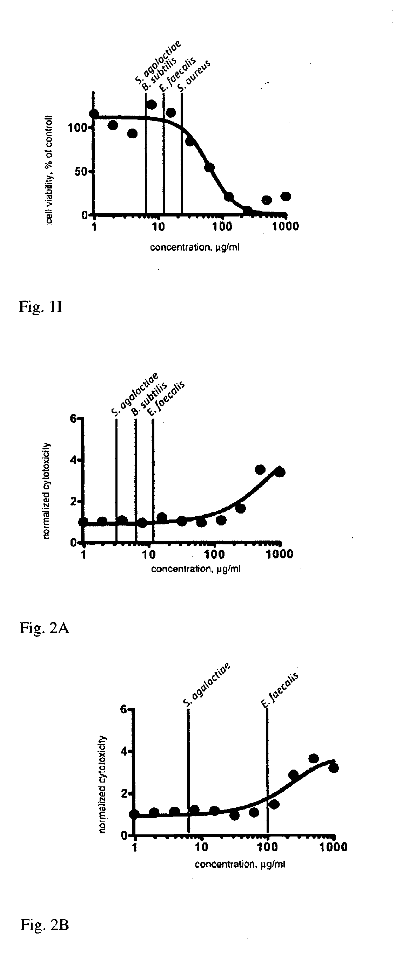

An effective inhibitor of *Giardia* trophozoite growth termed phosphonoxin (1) (Figure 1) with an activity that rivaled existing therapeutics was recently reported. Phosphonoxin was designed as a transient-state inhibitor of a glycosyl transferase - cyst wall synthase (CWS). CWS catalyzes synthesis of the chitin-like poly β-1-3-linked *N*-acetylgalactosamine [poly(GalNAc)] that comprises about 63% of the giardia cyst wall. Although phosphonoxin did not specifically inhibit cyst formation, it potently inhibited vegetative growth. (Suk D. H., Rejman D. et al., Bioorg. Med. Chem. Letters 2007, 17(10), 2811). The molecule of phosphonoxin display certain structural similarity to several types of nucleoside antibiotics: (i) polyoxins **3,** (ii) muraymycins **4,** and (iii) caprazamycins **5.**

The polyoxins are a new group of antifungal antibiotics isolated from *Streptomyces cacaoi* that inhibit the growth of a number of mycelial fungi by interfering with chitin synthesis. (Isono, K., Asahi K., Suzuki S., J. Am. Chem. Soc. 1969, 91, 7490; Isono K. et al., Agricultural Biol. Chem. 1965, 29, 848; Endo A., Kakiki K., Misato J., J. Bacteriol. 1970, 104, 189; Ohta N., Kakiki K., Misato, T., Agric. Biol. Chem. 1970, 34, 1224). Polyoxin D shares a gross structural similarity with uridine diphospho-*N*-acetyl-D-glucosamine (UDP-GlcNAc 2) in agreement with its role as a competitive inhibitor.

The muraymycins, isolated from a culture broth of *Streptomyces* species are members of a class of naturally occurring 6'-*N*-alkyl-5'-β-*O*-aminorybosyl-*C*-glycyluridine antibiotics. The muraymycins bearing lipophylic side chain exhibit excelent activity against gram-positive bacteria. The muraymycins inhibit the formation of lipid II and peptidoglycan and are believed to be inhibitors of phosphor-MurNAc-pentapeptide translocase (MraY), which is responsible for the formation of lipid I in the peptidoglycan biosynthesis pathway. ( Bugg T. D. H., Lloyd A. J., Roper D. I., Infect. Dis. Drug Targets 2006, 6, 85. Kimura K., Bugg T. D. H., Nat. Prod. Rep. 2003, 20, 252; Bouhss A. et al., J. Mol. Microbiol. 1999, 34, 576; Bouhss A., et al., FEMS Microbiol. Rev. 2008, 32, 208. Bouhss, A. et al., FEMS Microbiol. Rev. 2008, 32, 208). Tanino recently described the synthesis of lipophilic muraymycin analog **6** exhibiting a significant activity against methicillin-resistant *Staphylococcus aureus* (MRSA) and vancomycin-resistant *Enterococci* (VRE). (Tanino T. et al., Med. Chem. Lett. 2010, 1, 258).

The caprazamycins (CPZs) (**5**) were isolated from the culture broth of the actinomycete strain *Streptomyces* spp. MK730-62F2 in 2003 (Igarashi M. et al., J. Antibiot. 2003, 56, 580; Igarashi M. et al., J. Antibiot. 2005, 58, 327) and represent the most recent members of the class of naturally occurring 6'-*N*-alkyl-5'-β-*O*-aminoribosyl-*C*-glycyluridine antibiotics that include the liposidomycins (7) (LPMs). The CPZs have shown excellent antimycobacterial activity *in vitro* not only against drug-susceptible (MIC = 3.13 µg/mL) but also multidrug-resistant *Mycobacterium tuberculosis* strains (MIC = 3.13 µg/mL) and exhibit no significant toxicity in mice. The biological target of the 6'-*N-*alkyl-5'-β-*O*-aminoribosyl-*C*-glycyluridine class of antibiotics is believed to be MraY translocase (IC₅₀= 0.05 µg/mL for LPMs). (Kimura K. et al., Agric. Biol. Chem. 1989, 53, 1811)

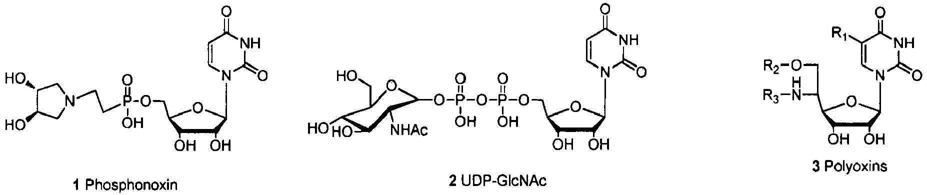

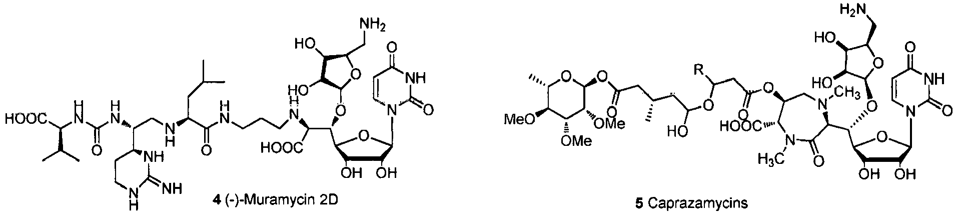

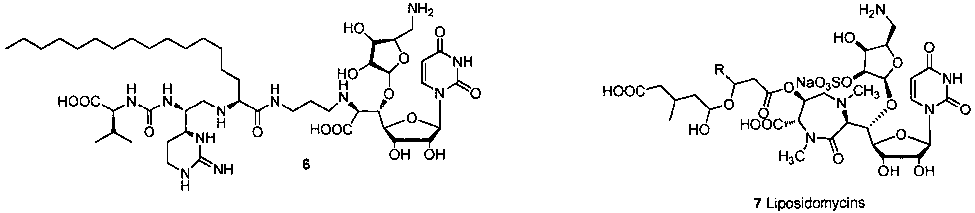

### Disclosure of the Invention

This invention provides novel compound of general formula I that exhibit significant antibacterial activity, in particular against gram-positive bacteria. The advantages of these compounds include simple synthesis and modular structure allowing for further optimization of their biological properties.

An object of this invention are lipophosphonoxins of general formula I 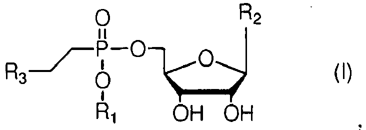
wherein R₁ is (C₈-C₂₂)alkyl or hexadecyloxypropyl, tetradecyloxypropyl, tetradecyloxyethyl, or hexadecyloxyethyl, and
R₂ is uracil, thymin or cytosin,
R₃ is selected from the group comprising compounds of general formula II and III: 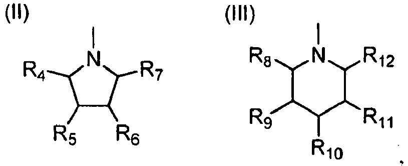
where R₄ is H or CH₂OH, R₅ is H or OH, R₆ is H or OH, R₇ is H or CH₂OH, R₈ is H or CH₂OH, R₉ is H or OH, R₁₀ is H or OH, R₁₁ is H or OH, R₁₂ is H or CH₂OH,
diastereomers and mixtures of diastereomers thereof, and
their pharmaceutically acceptable salts and hydrates.

Compounds of formula I contain several chiral centers (particularly on the phosphorus atom and in the R₃ group). The presence of a chiral center allows a compound to exist as one of two possible optical isomers ((R)- or (S)-enantiomer), or as a racemic mixture of both. In this case, where several chiral centers are present, diastereoisomers and mixtures of diastereoisomers are obtained, which are all covered by general formula I and included in the scope of this invention.

Another object of this invention is the synthesis of the novel lipophosphonoxins. The starting material for the synthesis of the final compounds arc nucleoside vinylphosphonic acids of general formula 11 (Scheme 2). These compounds are prepared by esterification of methyl vinylphosphonate **9** (Gao, Feng et al.; Chemistry-A European Journal, 2009, 15, 9, 2064 ― 2070) that is prepared according to literature procedures. The synthesis according to this invention is novel, simpler than the previously described procedures and affords good yields.
In the first step A), the comercially available dimethyl vinylphosphonate **VIII** (see Scheme 1) is heated overnight in 40% - 70% aqueous pyridine at 50 - 80 °C.

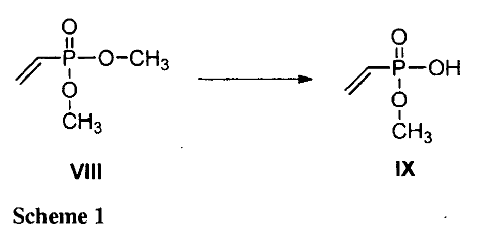

The obtained methyl vinylphosphonate **IX** is allowed to react in the second reaction step B with nucleoside **X** possessing free 5'-hydroxyl group and protected in the positions 2'and 3'with a group selected from 2',3'-isopropylidene, 2',3'-bis(dimethoxytrityl), 2',3'-dibenzoyl, and 2',3'-diacetyl.

The esterification is carried out using triisopropylbenzenesulfonylchloride (TPSCl) under *N-*methylimidazole catalysis in a solvent selected from the group comprising acetonitrile, dioxane, tetrahydrofuran (THF), dichloroethane, chloroform and dichloromethane. The obtained methyl ester is purified by chromatography on silica gel using linear gradient of ethanol in chloroform. The methyl ester group is subsequently removed by heating with 40% - 70% aqueous pyridine at 50 - 80 °C overnight (Scheme 2). The obtained vinylphosphonic acid **XI** is purified by chromatography on silica gel using linear gradient of mixture H1 (ethyl acetate:acetone:ethanol:water, volume ratios 4:1:1:1) in ethyl acetate.

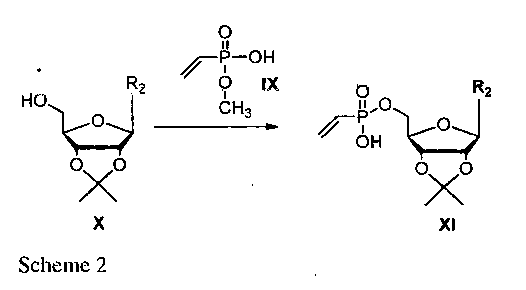

In the next step C), the vinylphosphonic acids **XI** are esterified with alcohol using TPSCl as a condensing agent under *N*-methylimidazole catalysis in a solvent selected from the group comprising acetonitrile, dioxane, tetrahydrofuran (THF), dichloroethane, chloroform and dichloromethane (Scheme 3). The obtained vinylphosphonate **XII** is purified by chromatography on silica gel using linear gradient of ethanol in chloroform.

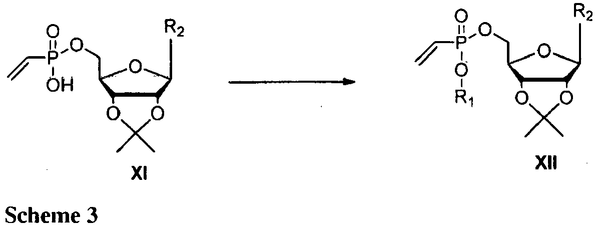

The final step D) consists in Michael addition of secondary amine to the vinyl moiety of intermediate **XII.** The reaction is accomplished by heating in a solvent selected from the group comprising acetonitrile, dioxane, THF, dichloroethane, chloroform, ethanol, propanol, isopropanol and n-butanol at 80 - 120 °C for 4 to 12 hours. Subsequently, the protecting groups are removed from the nucleoside moiety. The final product is isolated by chromatography on silica gel using linear gradient of mixture H1 (as described above) in ethyl acetate. If required, the final product is re-purified by preparative HPLC on reverse phase.

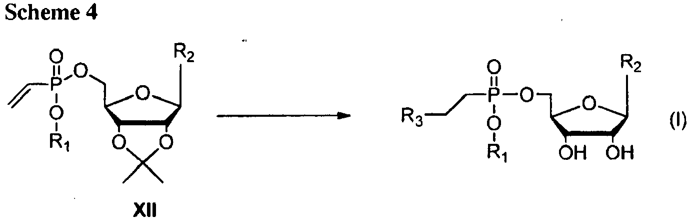

It is an aspect of the method of preparation of lipophosphonoxins of general formula I or their diastereomers or mixtures of such diastereomers, that heating overnight with aqueous pyridine in steps A) and/or B) is preferably carried out at 60 °C and the concentration of the aqueous pyridine solution is preferably 60 vol. %.

In another preferred embodiment, the Michael addition in the step D is carried out in n-butanol at 105 °C; more preferably, the reaction is perfomed overnight.

In one aspect of the present invention, the esterification in the steps B and/or C is carried out preferably in dichloromethane.

In another preferred embodiment of the method of the preparation of lipophosphonoxins of general formula I or their diastereomers and mixtures of such diastereomers, in the step D), the nucleoside protecting groups are removed by ethanolic methylamine or aqueous ammonia: if 2',3'-protected ribonucleoside is used, the protecting group is preferably removed by treatment with 0.5 mol.l⁻¹ hydrochloride in methanol overnight at room temperature.

Another object of the present invention are the lipophoshonoxins of general formula I or their diastereomers or pharmaceutically acceptable salts and hydrates thereof and/or mixtures of such compounds for use as medicaments.

Yet another object of the present invention are the lipophoshonoxins of general formula I or their diastereomers or pharmaceutically acceptable salts and hydrates thereof and/or mixtures of such compounds for use as antibacterial medicaments.

Another object of the present invention is an antibacterial medicament containing as the active ingredient at least one lipophoshonoxin of general formula I or their diastereomers or pharmaceutically acceptable salts and hydrates thereof and/or mixture of such compounds.

Finally, an object of the invention is the use of lipophoshonoxins of general formula I or their diastereomers or pharmaceutically acceptable salts and hydrates thereof and/or mixtures of such compounds as active components of disinfectants and/or selective growth media for *in vitro* cultivations.

The compounds according to this invention possess antibacterial activity particularly against bacterial strains *Enterococcus faecalis, Bacterium subtilis, Streptococcus agalactiae, Staphylococcus aureus, Staphylococcus haemolyticus, Enterococcus faecium, Staphylococcus epidermidis,* including strains resistant to current antibiotics.

The compounds according to this invention simultaneously exhibited none or very low effect on the viability of normal human erythroid progenitor cells cultivated *in vitro* in the range of concentrations sufficient for antibacterial activity. The same applies to cytotoxicity induced by these compounds.

The modular structure as well as the simple synthetic procedure consisting in connecting single modules allows for large structural variations of the compounds according to this invention that can lead to the modulation of their biological activity.

### Brief Description of the Figures

Figures 1A-I depict the cell viability determined by the commercially available CellTiter-Blue® Cell Viability Assay (Promega, catalogue. no. G8082). The x-axis represents concentration of the tested compound in µg/ml in logarithmic scale, the y-axis represents cell viability in % of control. 1A: compound from the example 9, 1B: compound from the example 10, 1C: compound from the example 11, 1D: compound from the example 12, 1E: compound from the example 13, 1F: compound from the example 14, 1G: compound from the example 15, 1H: compound from the example 16, 1I: compound from the example 17. For comparison, the antibacterial activities (MIC values) of these compounds against selected bacterial species are indicated with vertical lines in the charts.

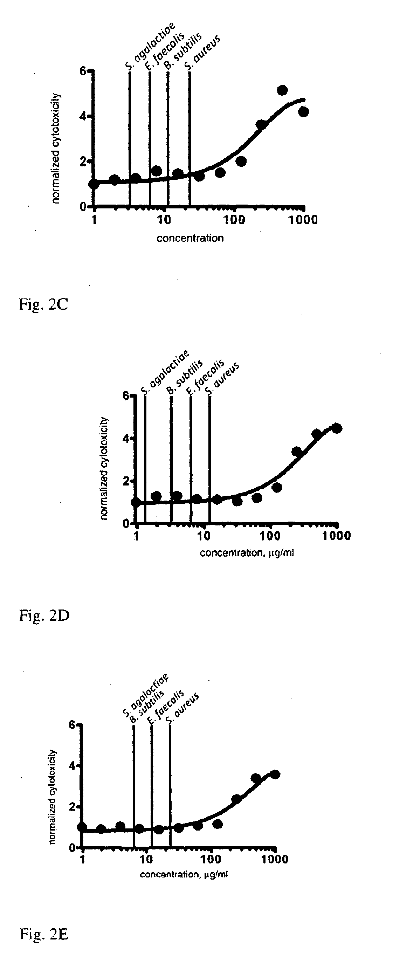

Figures 2A-E depict the cytotoxicity determined by the commercially available CytoTox-ONE™ Homogeneous Membrane Integrity Assay (Promega, catalogue no. G7892). The x-axis represents concentration of the tested compound in µg/ml in logarithmic scale, the y-axis represents normalized cytotoxicity described by a dimensionless number. 2A: compound from the example 9, 2B: compound from the example 14, 2C: compound from the example 15, 2D: compound from the example 16, 2E: compound from the example 17. For comparison, the antibacterial activities (MIC values) of these compounds against selected bacterial species are indicated with vertical lines in the charts.

### Examples

### Abbreviations:

- DCM: dichloromethane
- TPSCl: triisopropyl benzenesulfonylchloride
- IR: infrared spectrum
- HR-ESI: high resolution mass spectroscopy, using electrospray ionisation
- HR-EI: high resolution mass spectroscopy, using electron impact ionisation
- n-BuOH: n-butanol
- DMTr: dimethoxytrityl
- THF: tetrahydrofuran
- EC₅₀: half maximal effective concentration
- IC₅₀: half maximal inhibitory concentration

### A) Preparation of the novel compounds

### Example 1

### 2',3'-isopropylideneuridin-5'-yl-vinylphosphonate

To the mixture of the monomethyl phosphonate (9,15 g, 75 mmol), 2',3'-isopropylideneuridine (14 g, 50 mmol) and methylimidazole (5,9 ml, 150 mmol) in DCM (500 ml), TPSCl (45,43 g, 150 mmol) is added. The reaction mixture is stirred at room temperature (rt) overnight, then washed with a saturated aq NaHCO₃ (2 x 500 ml) followed by washing with 3% aq citric acid (2 x 500 ml) and dried over Na₂SO₄. Organic phase is concentrated *in vacuo* and monomethyl ester of nucleosidevinylphosphonic acid is obtained by flash chromatography on silica gel using linear gradient of ethanol in chloroform. The monomethyl ester intermediate in 40-70%, preferably in 60% (vol. %) aqueous pyridine (500 ml) is stirred at 50-80 °C, preferably at 60 °C overnight. The reaction mixture is concentrated *in vacuo,* co-evaporated in EtOH (2 x 300 ml), and dissolved in the same solvent (500 ml). Dowex 50 in Et₃N form (200 mll) is added and the suspension is stirred for 10 min. The resin is removed by filtration, and the filtrate is concentrated *in vacuo.* The title product is obtained by flash chromatography on silica gel using linear gradient of solvent mixture H1 (ethyl acetate:acetone:ethanol:water, volume ratios 4:1:1:1) in ethyl acetate in 48% yield (11.38 g, 23.93 mmol ) as a yellowish foam.

¹H NMR (499.8 MHz, CD₃OD): 1.35, 1.54 (2 × q, 2 × 3H, ⁴*J* = 0.5, (CH₃)₂C); 3.98 (dd, 2H, *J*_{H,P} = 5.3, *J*_{5',4'} = 3.4, H-5'); 4.35 (m, 1H, H-4'); 4.90 (m, 2H, H-2',3'); 5.74 (d, 1H, *J*₅,₆ = 8.1, H-5); 5.86 (ddd, 1H, *J*_{H,P} = 46.0, *J*_{cis} = 12.3, *J*_{gem} = 3.1, CH_{cis}**Hₜᵣₐₙₛ**=CHP); 5.95 (d, 1H, *J*_{1',2'} = 2.5, H-1'); 6.00 (ddd, 1H, *J*_{H,P} = 22.8, *J*ₜᵣₐₙₛ = 18.7, *J*_{gem} = 3.1, C**H_{cis}**Hₜᵣₐₙₛ=CHP); 6.12 (ddd, 1H, *J*_{H,P} = 19.7, *J*ₜᵣₐₙₛ = 18.7, *J*_{cis} = 12.3, =CHP); 7.86 (d, 1H, *J*_{6,5} = 8.1, H-6).

¹³C NMR (125.7 MHz, CD₃OD): 25.53, 27.55 ((CH₃)₂C); 65.31 (d, *J*_{C,P} = 4.8, CH₂-5'); 82.49 (CH-3'); 85.67 (CH-2'); 86.56 (d, *J*_{C,P} = 8.0, CH-4'); 93.40 (CH-1'); 102.98 (CH-5); 114.99 (C(CH₃)₂); 130.74 (CH₂=CHP); 132.70 (d, *J*_{C,P} = 174.3, =CHP); 143.42 (CH-6); 152.16 (C-2); 166.15 (C-4).

³¹P NMR (202.3 MHz, CD₃OD): 13.16.

**IR** νₘₐₓ(KBr) 3200 (w, br), 2977 (m), 2939 (m), 2803-2100 (s-vw), 1715 (s, sh), 1695 (vs), 1630 (w, sh), 1612 (w, sh), 1469 (m), 1434 (m), 1398 (m), 1384 (m), 1373 (m, sh), 1273 (m), 1214 (s), 1115 (m, sh), 1070 (s, br), 1037 (s), 1018 (m, sh), 995 (w), 765 (w), 517 (w) cm⁻¹.

**HR-ESI** C₁₄H₁₈O₈N₂P (M-H)⁻ calcd 373.0806; found 373.0809

### Example 2

### 2',3'-Isopropylideneuridin-5'-yl tetradecyl vinylphosphonate

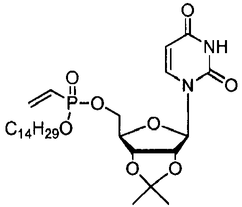

TPSCl (4.68 g, 15.45 mmol) is added to the mixture of vinylphosphonic acid from the example 1 (1.93 g, 5.15 mmol), tetradekanol (2.2 g, 10.31 mmol), and N-methylimidazole (1.22 ml, 15.45 mmol) in DCM (50 ml). The reaction mixture is stirred overnight at rt, then extracted/washed with saturated solution of NaHCO₃ (2 x 100 ml), 3% aqueous citric acid (2 x 100 ml) and dried over Na₂SO₄. The organic phase is concentrated *in vacuo* and the product is obtained by flash chromatography on silica gel using linear gradient of ethanol in chloroform in 40% yield (1.17 g, 2.05 mmol) in the form of yellowish viscous oil.
A mixture of diastereoisomers ~ 6:5

**¹H NMR** (499.8 MHz, CDCl₃): 0.88 (m, 6H, C**H₃**(CH₂)₁₃); 1.20-1.35 (m, 44H, CH₃(C**H₂**)₁₁CH₂CH₂₋O); 1.350, 1.354, 1.573, 1.576 (4 × q, 4 × 3H, ⁴*J* = 0.7, (CH₃)₂C); 1.67 (m, 4H, CH₃(CH₂)₁₁**CH**₂CH₂O); 4.03, 4.04 (2 × dt, 2 ^{×} 2H, *J*_{H,P} = 7.3, *J*_{vic} = 6.7, CH₃(CH₂)₁₁C**H₂**CH₂O); 4.19-4.29 (m, 4H, H-5'); 4.35-4.39 (m, 2H, H-4'); 4.85, 4.86 (2 × dd, 2 × 1H, *J*_{3',2'} = 6.4, *J*_{3',4'} = 3.6, H-3'); 4.88, 4.89 (2 × dd, 2 × 1H, *J*_{2',3'} = 6.4, *J*_{2',1'} = 2.3, H-2'); 5.71, 5.72 (2 × d, 2 × 1H, *J*_{5,6} = 8.1, H-5); 5.77, 5.81 (2 × d, 2 × 1H, *J*_{1',2'} = 2.3, H-1'); 6.03, 6.04 (2 × ddd, 2 × 1H, *J*_{H,P} = 22.9, *J*ₜᵣₐₙₛ = 18.6, *J*_{cis} = 12.7, =CHP); 6.16, 6.19 (2 × ddd, 2 × 1H, *J*_{H,P} = 51.6, *J*_{cis} = 12.7, *J*_{gem} = 2.0, CH_{cis}**Hₜᵣₐₙₛ**=CHP); 6.33, 6.34 (2 × ddd, 2 × 1H, *J*_{H,P} = 25.7, *J*ₜᵣₐₙₛ = 18.7, *J*_{gem} = 2.0, C**H_{cis}**Hₜᵣₐₙₛ=CHP); 7.38, 7.43 (2 × d, 2 × 1H, *J*_{6,5} = 8.1, H-6); 9.34 (bs, 2H, NH).
¹³**C NMR** (125.7 MHz, CDCl₃): 14.08 (CH₃(CH₂)₁₃); 22.64 (CH₃(CH₂)₁₁CH₂CH₂O); 25.23, 25.25 ((CH₃)₂C); 25.43 (CH₃(CH₂)₁₁CH₂CH₂O); 27.08, 27.09 ((CH₃)₂C); 29.10, 29.30, 29.46, 29.52, 29.59, 29.60, 29.62, 29.64 (CH₃(CH₂)₁₁CH₂CH₂O); 30.39 (d, *J*_{C,P} = 6.2, CH₃(CH₂)₁₁CH₂CH₂O); 31.87 (CH₃(CH₂)₁₁CH₂CH₂O); 64.93, 65.01 (d, *J*_{C,P} = 5.5, CH₂-5'); 66.47, 66.48 (d, *J*_{C,P} = 5.7, CH₃(CH₂)₁₁CH₂CH₂O); 80.57, 80.66 (CH-3'); 84.45, 84.53 (CH-2'); 85.26, 85.55 (d, *J*_{C,P} = 7.0, CH-4'); 93.43, 93.83 (CH-1'); 102.49, 102.58 (CH-5); 114.54, 114.59 (C(CH₃)₂); 124.97, 125.02 (d, *J*_{C,P} = 184.3, =CHP); 136.60, 136.62 (CH₂=CHP); 141.47, 141.55 (CH-6); 149.96, 149.99 (C-2); 163.11 (C-4).
**³¹P NMR** (202.3 MHz, CDCl₃): 18.59, 18.74.
**IR** *v*ₘₐₓ(CHCl₃) 3390 (w), 3170 (w, br), 2957 (s), 2928 (vs), 2856 (s), 1715 (vs), 1695 (vs), 1634 (m), 1616 (m), 1457 (s), 1421 (m), 1399 (m), 1384 (s), 1378 (s), 1240 (vs, br), 1121 (s), 1109 (s), 1070 (vs), 1011 (vs), 988 (s), 973 (s, sh), 860 (s), 544 (m), 512 (m) cm⁻¹.
**HR-ESI** C₂₈H₄₈O₈N₂P [M+H]⁺ calcd 571.31428, found 571.31445, C₂₈H₄₇O₈N₂NaP [M+Na]⁺ calcd 593.29622, found 593.29616.

### Example 3

### Hexadecyl-2',3'-isopropylideneuridin-5'-yl-vinylphosphonate

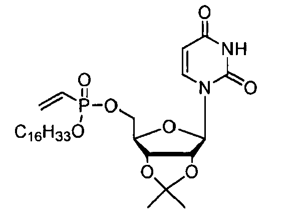

TPSCI (4.12 g, 13.62 mmol) is added to the mixture of vinylphosphonic acid from the example 1 (1.7 g, 4.54 mmol), hexadecanol (2.9 g, 12 mmol), and 1-methylimidazole (1.08 ml, 13.62 mmol) in DCM (50 ml). The reaction mixture is stirred overnight at rt, then extracted/washed with saturated solution of NaHCO₃ (2 x 100 ml), 3% aqueous citric acid (2 x 100 ml) and dried over Na₂SO₄. The organic phase is concentrated *in vacuo* and the product is obtained by flash chromatography on silica gel using linear gradient of ethanol in chloroform in 50% yield (1.38 g, 2.3 mmol) in the form of colorless viscous oil.

### A mixture of diastereoisomers ∼ 6:5

**¹H NMR** (499.8 MHz, CDCl₃): 0.88 (m, 6H, C**H₃**(CH₂)₁₅); 1.22-1.35 (m, 52H, CH₃(C**H₂**)₁₃CH₂CH₂₋O); 1.350, 1.354, 1.573, 1.577 (4 × q, 4 × 3H, ⁴*J* = 0.7, (CH₃)₂C); 1.67 (m, 4H, CH₃(CH₂)₁₃C**H₂**CH₂O); 4.03, 4.04 (2 × dt, 2 × 2H, *J*_{H,P} = 7.3, *J*_{vic} = 6.7, CH₃(CH₂)₁₃CH₂C**H₂**O); 4.19-4.30 (m, 4H, H-5'); 4.35-4.39 (m, 2H, H-4'); 4.85, 4.86 (2 × dd, 2 × 1H, *J*_{3',2'} = 6.4, *J*_{3',4'} = 3.6, H-3'); 4.878, 4.8984 (2 × dd, 2 × 1H, *J*_{2',3'} = 6.4, *J*_{2',1'} = 2.3, H-2'); 5.71, 5.72 (2 × dd, 2 × 1H, *J*_{5,6} = 8.1, *J*_{5,NH} = 2.2, H-5); 5.78, 5.81 (2 × d, 2 × 1H, *J*_{1',2'} = 2.3, H-1'); 6.03, 6.05 (2 × ddd, 2 × 1H, *J*_{H,P} = 22.8, *J*ₜᵣₐₙₛ = 18.6, *J*_{cis} = 12.8, =CHP); 6.12, 6.13 (2 × ddd, 2 × 1H, *J*_{H,P} = 51.7, *J*_{cis} = 12.8, *J*_{gem} = 2.0, CH_{cis}**Hₜᵣₐₙₛ**=CHP); 6.33, 6.34 (2 × ddd, 2 × 1H, *J*_{H,P} = 24.8, *J*ₜᵣₐₙₛ = 18.6, *J*_{gem} = 2.0, C**H_{cis}**Hₜᵣₐₙₛ=CHP); 7.38, 7.43 (2 × d, 2 × 1H, *J*_{6,5} = 8.1, H-6); 9.15, 9.18 (2 × bs, 2 × 2H, NH).

**¹³C NMR** (125.7 MHz, CDCl₃): 14.08 (CH₃(CH₂)₁₅); 22.65 (CH₃(CH₂)₁₃CH₂CH₂O); 25.23, 25.26 ((CH₃)₂C); 25.43 (CH₃(CH₂)₁₃CH,CH₂O); 27.08, 27.10 ((CH₃)₂C); 29.11, 29.32, 29.47, 29.53, 29.60, 29.61, 29.63, 29.65 (CH₃(CH₂)₁₃CH₂CH₂O); 30.40 (d, *J*_{C,P} = 6.3, CH₃(CH₂)₁₃CH₂CH₂O); 31.88 (CH₃(CH₂)₁₃CH₂CH₂O); 64.93, 65.00 (d, *J*_{C,P} = 5.5, CH₂-5'); 66.47, 66.48 (d, *J*_{C,P} = 5.7, CH₃(CH₂)₁₃CH₂CH₂O); 80.57, 80.66 (CH-3'); 84.46, 84.54 (CH-2'); 85.25, 85.55 (d, *J*_{C,P} = 7.1, CH-4'); 93.40, 93.80 (CH-1'); 102.49, 102.57 (CH-5); 114.55, 114.60 (C(CH₃)₂); 124.99, 125.04 (d, *J*_{C,P} 184.2, =CHP); 136.62, 136.66 (d, *J*_{C,P} = 2.0, CH₂=CHP); 141.46, 141.55 (CH-6); 149.92, 149.95 (C-2); 163.05, 163.07 (C-4).

**³¹P NMR** (202.3 MHz, CDCl₃): 18.59, 18.73.

**HR-ESI** C₃₀H₅₁O₈N₂PNa (M+Na)⁺ calcd 621.32752; found 621.32778

### Example 4

### 2',3'-Isopropylidenuridin-5'-yl octadecyl vinylphosphonate

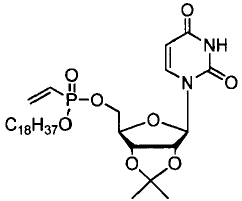

TPSCl (4.16 g, 13.74 mmol) is added to the mixture of vinylphosphonic acid from the example 1 (1.7 g, 4.54 mmol), octadecanol (2.48 g, 9.17 mmol), and 1-methylimidazole (1.08 ml, 13.74 mmol) in DCM (50 ml). The reaction mixture is stirred overnight at rt, then extracted/washed with saturated solution of NaHCO₃ (2 x 100 ml), 3% aqueous citric acid (2 x 100 ml) and dried over Na₂SO₄. The rganic phase is concentrated *in vacuo* and the product is obtained by flash chromatography on silica gel using linear gradient of ethanol in chloroform in 43% yield (1,23 g, 1,96 mmol) in the form of colorless viscous oil.

### A mixture of diastereoisomers ∼ 6:5

**¹H NMR** (499.8 MHz, CDCl₃): 0.88 (m, 6H, C**H₃**(CH₂)₁₇); 1.20-1.40 (m, 60H, CH₃(C**H₂**)₁₅CH₂CH₂₋O); 1.350, 1.354, 1.573, 1.576 (4 × q, 4 × 3H, ⁴*J* = 0.7, (CH₃)₂C); 1.66 (m, 4H, CH₃(CH₂)₁₅C**H₂**CH_{2O}); 4.03, 4.04 (2 × dt, 2 × 2H, *J*_{H,P} = 7.3, *J*_{vic} = 6.7, CH₃(CH₂)₁₅CH₂C**H₂**O); 4.19-4.29 (m, 4H, H-5'); 4.34-4.39 (m, 2H, H-4'); 4.85, 4.86 (2 × dd, 2 × 1H, *J*_{3',2'} = 6.4, *J*_{3',4'} = 3.7, H-3'); 4.88, 4.89 (2 × dd, 2 × 1H, *J*_{2'},_{3'} = 6.4, *J*_{2',1'} = 2.3, H-2'); 5.71, 5.72 (2 × d, 2 × 1H, *J*_{5,6} = 8.1, H-5); 5.77, 5.81 (2 × d, 2 × 1H, *J*_{1'},_{2'} = 2.3, H-1'); 6.03, 6.04 (2 × ddd, 2 × 1H, *J*_{H,P} = 22.9, *J*ₜᵣₐₙₛ = 18.6, *J*_{cis} = 12.7, =CHP); 6.16, 6.19 (2 × ddd, 2 × 1H, *J*_{H,P} = 51.7, *J*_{cis} = 12.7, *J*_{gem} = 2.0, CH_{cis}**Hₜᵣₐₙₛ**=CHP); 6.33, 6.34 (2 × ddd, 2 × 1H, *J*_{H,P} = 25.6, *J*ₜᵣₐₙₛ = 18.6, *J*_{gem} = 2.0, C**H_{cis}**Hₜᵣₐₙₛ=CHP); 7.38, 7.43 (2 × d, 2 × 1H, *J*_{6,5} = 8.1, H-6); 9.37 (bs, 2H, NH).

**¹³C NMR** (125.7 MHz, CDCl₃): 14.08 (CH₃(CH₂)₁₇); 22.64 (CH₃(CH₂)₁₅CH₂CH₂O); 25.22, 25.25 ((CH₃)₂C); 25.43 (CH₃(CH₂)₁₅CH₂CH₂O); 27.07, 27.09 ((CH₃)₂C); 29.10, 29.31, 29.46, 29.53, 29.60, 29.61, 29.63, 29.65 (CH₃(CH₂)₁₅CH₂CH₂O); 30.39 (d, *J*_{C,P} = 6.2, CH₃(CH₂)₁₅CH₂CH₂O); 31.87 (CH₃(CH₂)_{15C}H₂CH₂O); 64.93, 65.01 (d, *J*_{C,P} = 5.5, CH₂-5'); 66.46, 66.47 (d, *J*_{C,P} = 5.7, CH₃(CH₂)₁₅CH₂CH₂O); 80.57, 80.66 (CH-3'); 84.45, 84.53 (CH-2'); 85.27, 85.56 (d, *J*_{C,P} = 7.0, CH-4'); 93.44, 93.83 (CH-1'); 102.49, 102.58 (CH-5); 114.53, 114.58 (C(CH₃)₂); 124.97, 125.01 (d, *J*_{C,P} = 184.2, =CHP); 136.62, 136.66 (d, *J*_{C,P} = 2.0, CH₂=CHP); 141.47, 141.56 (CH-6); 149.97, 150.00 (C-2); 163.14, 163.16 (C-4).

**³¹P NMR** (202.3 MHz, CDCl₃): 18.59, 18.74.

**IR** *v*ₘₐₓ(CHCl₃) 3390 (w), 3170 (w, br), 2928 (vs), 2855 (vs), 1716 (vs), 1695 (vs), 1634 (m), 1615 (w), 1457 (s), 1420 (m), 1399 (m), 1385 (s), 1378 (s), 1246 (s, br), 1121 (s), 1110 (s), 1071 (s), 1010 (s), 988 (s), 974 (s, sh), 860 (m), 544 (w), 512 (w) cm⁻¹.

**HR**-ESI C₃₂ H₅₆ O₈ N₂ P [M+H]⁺ calcd 627.37688, found 627.37738.

### Example 5

### Icosanyl 2',3'-isopropylideneuridin-5'-yl vinylphosphonate

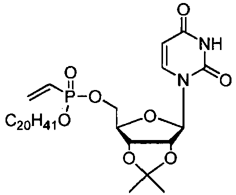

TPSCl (4.49 g, 14.82 mmol) is added to the mixture of vinylphosphonic acid from the example 1 (2.35 g, 4.94 mmol), icosanol (2,95 g, 9,9 mmol), and 1-methylimidazole (1.17 ml, 14.82 mmol) in DCM (50 ml). The reaction mixture is stirred overnight at rt, then extracted/washed with saturated solution of NaHCO₃ (2 x 100 ml), 3% aqueous citric acid (2 x 100 ml) and dried over Na₂SO₄. The organic phase is concentrated *in vacuo* and the product is obtained by flash chromatography on silica gel using linear gradient of ethanol in chloroform in 34% yield (1,1 g, 1,68 mmol) in the form of colorless wax.

### A mixture of diastereoisomers ∼ 6:5

**¹H NMR** (499.8 MHz, CDCl₃): 0.88 (m, 6H, C**H₃**(CH₂)₁₉); 1.20-1.36 (m, 68H, CH₃(C**H₂**)₁₇CH₂CH₂₋O); 1.351, 1.354, 1.574, 1.577 (4 × q, 4 × 3H, ⁴*J* = 0.7, (CH₃)₂C); 1.66 (m, 4H, CH₃(CH₂)₁₇C**H₂**CH₂O); 4.03, 4.04 (2 × dt, 2 × 2H, *J*_{H,P} = 7.3, *J*_{vic} = 6.7, CH₃(CH₂)₁₇CH₂C**H₂**O); 4.19-4.29 (m, 4H, H-5'); 4.35-4.39 (m, 2H, H-4'); 4.84, 4.86 (2 × dd, 2 × 1H, *J*_{3',2'} = 6.4, *J*_{3',4'} = 3.4, H-3'); 4.87, 4.89 (2 × dd, 2 × 1H, *J*_{2',3'} = 6.4, *J*_{2',1'} = 2.3, H-2'); 5.706, 5.714 (2 × d, 2 × 1H, *J*_{5,6} = 8.1, H-5); 5.77, 5.80 (2 × d, 2 × 1H, *J*_{1',2'} = 2.3, H-1'); 6.03, 6.06 (2 × ddd, 2 × 1H, *J*_{H,P} = 22.8, *J*ₜᵣₐₙₛ = 18.6, *J*_{cis} = 12.7, =CHP); 6.17, 6.19 (2 × ddd, 2 × 1H, *J*_{H,P} = 51.7, *J*_{cis} = 12.7, *J*_{gem} = 2.0, CH_{cis}**Hₜᵣₐₙₛ**=CHP); 6.33, 6.34 (2 × ddd, 2 × 1H, *J*_{H,P} = 25.6, *J*ₜᵣₐₙₛ = 18.6, *J*_{gem} = 2.0, C**H_{cis}**Hₜᵣₐₙₛ=CHP); 7.37, 7.43 (2 × d, 2 × 1H, *J*_{6,5} = 8.1, H-6); 9.08 (bs, 2H, NH).

**¹³C NMR** (125.7 MHz, CDCl₃): 14.10 (CH₃(CH₂)₁₉); 22.66 (CH₃(CH₂)₁₇CH₂CH₂O); 25.24, 25.26 ((CH₃)₂C); 25.44 (CH₃(CH₂)₁₇CH₂CH₂O); 27.09, 27.11 ((CH₃)₂C); 29.12, 29.33, 29.48, 29.55, 29.62, 29.65, 29.67 (CH₃(CH₂)₁₇CH₂CH₂O); 30.41 (d, *J*_{C,P} = 6.3, CH₃(CH₂)₁₇CH₂CH₂O); 31.89 (CH₃(CH₂)₁₇CH₂CH₂O); 64.92, 64.99 (d, *J*_{C,P} = 5.5, CH₂-5'); 66.48, 66.50 (d, *J*_{C,P} = 5.7, CH₃(CH₂)₁₇CH₂CH₂O); 80.57, 80.66 (CH-3'); 84.47, 84.56 (CH-2'); 85.25, 85.54 (d, *J*_{C,P} = 7.0, CH-4'); 93.44, 93.83 (CH-1'); 102.50, 102.59 (CH-5); 114.56, 114.62 (C(CH₃)₂); 124.98, 125.02 (d, *J*_{C,P} = 184.2, =CHP); 136.65, 136.70 (d, *J*_{C,P} = 1.9, CH₂=CHP); 141.41, 141.50 (CH-6); 149.90, 149.93 (C-2); 162.92, 162.95 (C-4).

**³¹P NMR** (202.3 MHz, CDCl₃): 18.59, 18.74.

IR *v*ₘₐₓ(CHCl₃) 3389 (w), 3167 (w, vbr), 2928 (vs), 2855 (s), 1715 (vs), 1695 (vs), 1634 (m), 1615 (w), 1457 (s), 1420 (w), 1399 (m), 1384 (s), 1378 (s), 1249 (s, sh), 1122 (m), 1110 (s), 1078 (s), 1011 (s), 989 (s), 975 (s, sh), 860 (m), 544 (w), 512 (w) cm⁻¹.

**HR-ESI** C₃₄H₆₀O₈N₂P [M+H] calcd 655.40818, found 655.40857, C₃₄H₅₉O₈N₂NaP [M+Na] calcd 677.39012, found 677.39032.

### Example 6

### Hexadecyloxypropyl 2',3'-isopropylideneuridin-5'-yl vinyphosphonate

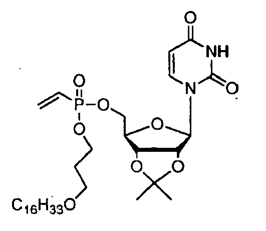

TPSCl (5.54 g, 18.3 mmol) is added to the mixture of vinylphosphonic acid from the example 1 (1.8 g, 6.1 mmol), hexadecyloxypropanol (1.45 g, 3.05 mmol), and 1-methylimidazole (1.45 ml, 18.3 mmol) in DCM (60 ml). The reaction mixture is stirred overnight at rt, then extracted/washed with saturated solution of NaHCO₃ (2 x 100 ml), 3% aqueous citric acid (2 x 100 ml) and dried over Na₂SO₄. The organic phase is concentrated *in vacuo* and the product is obtained by flash chromatography on silica gel using linear gradient of ethanol in chloroform in 71% yield (1.42 g, 2.16 mmol) in the form of colorless wax.
A mixture of diastereoisomers ~ 1:1
**¹H NMR** (500.0 MHz, CDCl₃): 0.88 (m, 6H, C**H₃**(CH₂)₁₅); 1.23-1.33 (m, 52H, CH₃(C**H₂**)₁₃CH₂CH₂₋O); 1.348, 1.352 (2 × q, 2 × 3H, ⁴*J*= 0.6, (CH₃)₂C); 1.54 (m, 4H, CH₃(C**H₂**)₁₃CH₂CH₂O); 1.57 (s, 6H, (CH₃)₂C); 1.92, 1.94 (2 × p, 2 × 2H, *J*_{vic} = 6.1, OCH₂C**H₂**CH₂OC₁₆H₃₃); 3.38, 3.39 (2 × t, 2 × 2H, *J*_{vic}, = 6.7, CH₃(CH₂)₁₃C**H₂**CH₂O); 3.48, 3.49 (2 × t, 2 × 2H, *J*_{vic} = 6.1, OCH₂CH₂CH₂OC₁₆H₃₃); 4.13, 4.15 (2 × td, 2 × 2H, *J*_{vic} = 6.1, *J*_{H,P} = 4.8, OC**H₂**CH₂CH₂OC₁₆H₃₃); 4.20-4.30 (m, 4H, H-5'); 4.34-4.38 (m, 2H, H-4'); 4.85, 4.86 (2 × dd, 2 × 1H, *J*_{3',2'} = 6.5, *J*_{3',4'} = 3.6, H-3'); 4.92 (dd, 2H, *J*_{2',3'} = 6.5, *J*_{2',1'} = 2.3, H-2'); 5.700, 5.704 (2 × d, 2 × 1H, *J*_{5,6} = 8.1 H-5); 5.74, 5.76 (2 × d, 2 × 1H, *J*_{1',2'} = 2.3, H-1'); 6.03, 6.05 (2 × ddd, 2 × 1H, *J*_{H,P} = 22.9, *J*ₜᵣₐₙₛ = 18.4, *J*_{cis} = 12.7, =CHP); 6.16, 6.18 (2 × ddd, 2 × 1H, *J*_{H,P} = 51_{.}8, *J*_{cis} = 12.7, *J*_{gem} = 1.9, CH_{cis}**Hₜᵣₐₙₛ**=CHP); 6.33, 6.35 (2 × ddd, 2 × 1H, *J*_{H,P} = 25.5, *J*ₜᵣₐₙₛ = 18.4, *J*_{gem} = 1.9, C**H_{cis}**Hₜᵣₐₙₛ=CHP); 7.34, 7.39 (2 × d, 2 × 1H, *J*_{6,5} = 8.1, H-6).
¹³**C NMR** (125.7 MHz, CDCl₃): 14.07 (CH₃(CH₂)₁₅); 22.62 (CH₃(CH₂)₁₃CH₂CH₂O); 25.19, 25.21 ((CH₃)₂C); 26.08 (CH₃(CH₂)₁₄CH₂O); 27.04, 27.06 ((CH₃)₂C); 29.29, 29.45, 29.55, 29.57, 29.58, 29.63 (CH₃(CH₂)₁₄CH₂O); 30.66, 30.68 (d, *J*_{C,P} = 6.4, OCH₂CH₂CH₂OC₁₆H₃₃); 31.85 (CH₃(CH₂)₁₃CH₂CH₂O); 63.56 63.57 (d, *J*_{C,P} = 5.5, OCH₂CH₂CH₂OC₁₆H₃₃); 64.92, 65.01 (d, *J*_{C,P} = 5.5, CH₂-5'); 66.32, 66.36 (OCH₂CH₂CH₂OC₁₆H₃₃); 71.15, 71.16 (CH₃(CH₂)₁₄CH₂O); 80.60, 80.67 (CH-3'); 84.44, 84.53 (CH-2'); 85.38, 85.62 (d, *J*_{C,P} = 7.1, CH-4'); 93.82, 94.12 (CH-1'); 102.57, 102.64 (CH-5); 114.42, 114.46 (C(CH₃)₂); 124.75, 124.80 (d, *J*_{C,P} = 184.0, =CHP); 136.75, 136.77 (d, *J* _{C,P} = 1.9, CH₂=CHP); 141.43, 141.48 (CH-6); 150.16 (C-2); 163.29, 163.32 (C-4).
³¹**P NMR** (202.3 MHz, CDCl₃): 18.64, 18.80.
**IR** vₘₐₓ(KBr) 2925 (vs), 2854 (s), 1709 9vs, sh), 1696 (vs), 1630 (w), 1459 (m), 1421 (m), 1400 (w, sh), 1381 (m), 1270 (m, sh), 1250 (m, sh), 1109 (s), 1078 (s), 1028 (s), 1012 (s), 972 (m, sh), 859 (m), 762 (w), 548 (w), 514 (w) cm⁻¹.
**HR-ESI** C₃₃H₅₈O₉N₂P (M+H)⁺ calcd 657.3874; found 657.3876

### Example 7

### 4-N-Benzoyl-2',3'-isopropylidenecytidin-5'-yl-vinylphosphonate

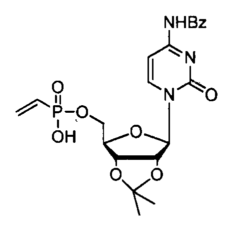

TPSCl (7.15 g, 23.61 mmol) is added to the mixture of monomethyl vinylphosphonate (1.95 g, 16 mmol), 4-*N*-benzoyl-2',3'-isopropylidenecytidine (3.05g, 7.87 mmol), and 1-methylimmidazole (1.9 ml, 23.61 mmol) in DCM (80 ml). The reaction mixture is stirred overnight at rt, then extracted/washed with saturated solution of NaHCO₃ (2 x 100 ml), 3% aqueous citric acid (2 x 100 ml) and dried over Na₂SO₄. Organic phase is concentrated *in vacuo* and monomethyl intermediate obtained by flash chromatography on silica gel using linear gradient of ethanol in chloroform is without detailed characterisation stirred in 40-70%, preferably in 60% (vol. %) aqueous pyridine (100 ml) at 50-80 °C, preferably at 60 °C, overnight. The reaction mixture is concentrated *in vacuo,* co-evaporated in EtOH (2 x 100 ml), and dissolved in the same solvent (100 ml). Dowex 50 in Et₃N form (80 ml) is added and the suspension is stirred for 10 min. The resin is removed by filtration, and the filtrate is concentrated *in vacuo.* The title product is obtained by flash chromatography on silica gel using linear gradient of solvent mixture H1 (ethyl acetate:acetone:ethanol: water, volume ratios 4:1:1:1) in ethyl acetate in 52% yield (1.95 g, 4.1 mmol) as a beige foam. NMR of 9b has shown Et₃NH⁺ salt (not free acid):
¹**H NMR** (600.1 MHz, CD₃OD): 1.31 (t, 9H, *J*_{vic} = 7.3, C**H₃**CH₂N); 1.37, 1.57 (2 × q, 2 × 3H, ⁴*J* = 0.7, (CH₃)₂C); 3.20 (q, 6H, *J*_{vic} = 7.3, CH₃C**H₂**N); 4.02 (ddd, 1H, *J*_{gem} = 11.5, *J*_{H,P} = 6.0, *J*_{5'b,4'} = 3.4, H-5'b); 4.06 (ddd, 1H, *J*_{gem} = 11.5, *J*_{H,P} = 4.5, *J*_{5'a,4'} = 3.0, H-5'a); 4.50 (m, 1H, H-4'); 4.91 (dd, 1H, *J*_{3',2'} = 6.0, *J*_{3',4'} = 2.2, H-3'); 4.93 (dd, 1H, *J*_{2',3'} = 6.0, *J*_{2',1'} = 2.3, H-2'); 5.86 (ddd, 1H, *J*_{H,P} = 46.0, *J*_{cis} = 12.4, *J*_{gem} = 3.1, CH_{cis}**Hₜᵣₐₙₛ**=CHP); 6.00 (d, 1H, *J*_{1',2'} = 2.3, H-1'); 6.01 (ddd, 1H, *J*_{H,P} = 22.9, *J*ₜᵣₐₙₛ = 18.7, *J*_{gem} = 3.1, C**H_{cis}**Hₜᵣₐₙₛ=CHP); 6.11 (ddd, 1H, *J*_{H,P} = 19.6, *J*ₜᵣₐₙₛ = 18.7, *J*_{cis} = 12.4, =CHP); 7.55 (m, 2H, H-*m*-Ph); 7.62 (bd, 1H, *J*_{5,6} = 7.5, H-5) 7.64 (m, 1H, H-*p*-Ph); 7.98 (m, 2H, H-*o*-Ph); 8.35 (d, 1H, *J*_{6,5} = 7.5, H-6).
¹³C NMR (150.9 MHz, CD₃OD): 9.18 (CH₃CH₂N); 25.45, 27.47 ((CH₃)₂C); 47.73 (CH₃CH₂N); 65.09 (d, *J*_{C,P} = 4.8, CH₂-5'); 82.47 (CH-3'); 87.16 (CH-2'); 87.91 (d, *J*_{C,P} = 8.1, CH-4'); 95.59 (CH-1'); 98.54 (CH-5); 114.69 (C(CH₃)₂); 129.16 (CH-*o*-Ph); 129.82 (CH-*m*-Ph); 130.80 (CH₂=CHP); 132.71 (d, *J* _{C,P} = 174.8, =CHP); 134.05 (CH-*p*-Ph); 134.78 (C-*i*-Ph); 147.24 (CH-6); 157.90 (C-2); 164.99 (C-4); 169.02 (CO).
³¹**P NMR** (202.3 MHz, CD₃OD): 13.02.
**HR-ESI** C₂₁H₂₃O₈N₃P (M-H)⁺ calcd 476.1223; found 476.1221.

### Example 8

### 4-N-Benzoyl-2',3'-isopropylidenecytidin-5'-yl hexadecyloxypropyl vinylphosphonate

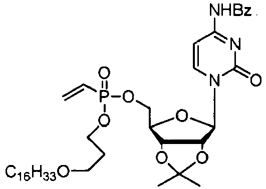

TPSCl (1.24 g, 4.08 mmol) is added to the mixture of vinylphosphonic acid from the example 7 (0.65 g, 1.36 mmol), hexadecyloxypropanol (0.81 g, 2.7 mmol), and 1-methylimidazole (1.45 ml, 18.3 mmol) in DCM (60 ml). The reaction mixture is stirred overnight at rt, then extracted/washed with saturated solution of NaHCO₃ (2 x 100 ml), 3% aqueous citric acid (2 x 100 ml) and dried over Na₂SO₄. Organic phase is concentrated *in vacuo* and product is obtained by flash chromatography on silica gel using linear gradient of ethanol in chloroform in 78% yield (0.81 g, 1.06 mmol) in the form of colorless wax.

### A mixture of diastereoisomers ∼ 1:1

**¹H NMR** (600.1 MHz, CD₃OD): 0.895 (m, 6H, C**H₃**(CH₂)₁₅); 1.24-1.35 (m, 52H, CH₃(C**H₂**)₁₃CH₂CH₂O); 1.368, 1.371 (2 × q, 2 × 3H, ⁴*J* = 0.6, (CH₃)₂C); 1.51 (m, 4H, CH₃(CH₂)₁₃C**H₂**CH₂O); 1.57 (s, 6H, (CH₃)₂C); 1.89, 1.91 (2 × p, 2 × 2H, *J*_{vic} = 6.1, OCH₂C**H₂**CH₂OC₁₆H₃₃); 3.38, 3.39 (2 × t, 2 × 2H, *J*_{vic} = 6.7, CH₃(CH₂)₁₃CH₂C**H₂**O); 3.47 (td, 2H, *J*_{vic} = 6.1, *J*_{H,P} = 1.2, OCH₂CH₂C**H₂**OC₁₆H₃₃); 3.49 (t, 2H, *J*_{vic} = 6.1, OCH₂CH₂C**H₂**OC₁₆H₃₃); 4.09-4.16 (m, 4H, OC**H₂**CH₂CH₂OC₁₆H₃₃); 4.27-4.36 (m, 4H, H-5'); 4.48 (m, 2H, H-4'); 4.91 (dd, 2H, *J*_{3'},_{2'} = 6.2, *J*_{3',4'} = 3.6, H-3'); 5.06, 507 (2 × dd, 2 × 1H, *J*_{2',3'} = 6.2, *J*_{2',1'} = 1.8, H-2'); 5.87, 5.88 (2 × d, 2 × 1H, *J*_{1',2'} = 1.8, H-1'); 6.145, 6.17 (2 × ddd, 2 × 1H, *J*_{H,P} = 24.0, *J*ₜᵣₐₙₛ = 18.4, *J*_{cis} = 12.8, =CHP); 6.257, 6.261 (2 × ddd, 2 × 1H, *J*_{H,P} = 52.0, *J*_{cis} = 12.8, *J*_{gem} = 2.1, CH_{cis}**Hₜᵣₐₙₛ**=CHP); 6.30, 6.31 (2 × ddd, 2 × 1H, *J*_{H,P} = 26.0, *J*ₜᵣₐₙₛ = 18.4, *J*_{gem} = 2.1, C**H_{cis}**Hₜᵣₐₙₛ=CHP); 7.54 (m, 4H, H-m-Bz); 7.59 (d, 2H, *J*_{5,6} = 7.7, H-5); 7.64 (m, 2H, H-p-Bz); 7.99 (m, 4H, H-o-Bz); 8.16, 8.17 (2 × d, 2 × 1H, *J*_{6,5} = 7.7, H-5).

**¹³C NMR** (150.9 MHz, CD₃OD): 14.48 (CH₃(CH₂)₁₅); 23.75 (CH₃(CH₂)₁₃CH₂CH₂O); 25.46, 25.48 ((CH₃)₂C); 27.27 (CH₃(CH₂)₁₄CH₂O); 27.43 ((CH₃)₂C); 30.49, 30.61, 30.78, 30.80, 30.81 (CH₃(CH₂)₁₄CH₂O); 31.70 (d, *J*_{C,P} = 6.5, OC**H₂**CH₂CH₂OC₁₆H₃₃); 33.09 (CH₃(CH₂)₁₃CH₂CH₂O); 64.96, 64.98 (d, *J*_{C,P} = 5.7, OCH₂CH₂CH_{2O}C₁₆H₃₃); 66.97, 67.04 (d, *J*_{C,P} = 5.6, CH₂-5'); 67.42, 67.43 (OCH₂CH₂CH₂OC₁₆H₃₃); 72.10 (CH₃(CH₂)₁₄CH₂O); 82.64, 82.635, 82.644 (CH-3'); 86.57, 86.58 (CH-2'); 88.23, 88.30 (d, *J*_{C,P} = 7.2, CH-4'); 97.60, 97.71 (CH-1'); 98.51, 98.54 (CH-5); 115.10, 115.11 (C(CH₃)₂); 125.63, 125.67 (d, *J*_{C,P} = 183.9, =CHP); 129.25 (CH-*o*-Bz); 129.83 (CH-*m*-Bz); 134.14 (CH-*p*-Bz); 134.70 (C-*i*-Bz); 138.01, 138.04 (d, *J*_{C,P} = 1.9, CH₂=CHP); 148.26, 148.34 (CH-6); 157.58, 157.60 (C-2); 165.58 (C-4); 169.19 (CO-Bz).

³¹ **P NMR** (202.3 MHz, CD₃OD): 19.62, 19.72.
**IR** *v*ₘₐₓ(CHCl₃) 3406 (w), 2928 (s), 2856 (m), 1703 (m), 1670 (s), 1628 (m), 1603 (w), 1554 (m), 1582 (w), 1497 (m, sh), 1480 (vs), 1455 (w, sh), 1400 (m), 1385 (m), 1377 (m), 1301 (m), 1248 (s, sh), 1186 (w), 1158 (m), 1121 (m), 1110 (m), 1078 (s), 1070 (s), 1028 (m), 1003 (m, sh), 989 (m), 909 (w), 865 (w), 842 (vw), 708 (w), 687 (w), 512 (w) cm⁻¹.
**HR-EI** C₄₀H₆₂N₃O₉P (M+H)⁺ calcd 759.4224; found 759.4225

### Example 9

### Hexadecyloxypropyl uridin-5'-yl 2-([3R,4R]-3,4-dihydroxypyrrolidin-1-N-yl)ethyl-phosphonate

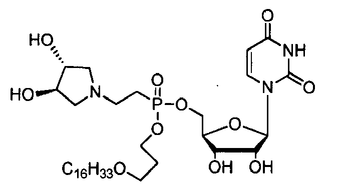

The mixture of (3*R*,4*R*)-3,4-dihydroxypyrrolidine (0.12 g, 1.18 mmol) and vinylphosphonate from the example 6 (0.4 g, 0.61 mmol) in n-BuOH (15 ml) is stirred at 105 °C overnight. The solvent is removed and the isopropylidene intermediate is obtained by flash chromatography on silica gel using linear gradient of H1 in ethyl acetate. This compound is dissolved in 0.5 M methanolic HCl (30 ml) and the mixture is stirred at rt for 4 h. The final product is obtained by flash chromatography on silica gel using linear gradient of H1 in ethyl acetate in in 34% overall yield (152 mg, 0.205 mmol) and lyophilized from water. A mixture of diastereoisomers ∼ 6:5
**¹H NMR** (499.8 MHz, CD₃OD): 0.90 (m, 6H, C**H**₃(CH₂)₁₅); 1.25-1.38 (m, 52H, CH₃(C**H**₂)₁₃CH₂CH₂-O); 1.55 (m, 4H, CH₃(CH₂)₁₃C**H₂**CH₂O); 1.93 (m, 4H, OCH₂C**H₂**CH₂OC₁₆H₃₃); 2.12-2.25 (m, 4H, CH₂P); 2.71 (dd, 4H, *J*_{gem} = 10.6, *J*_{vic} = 3.0, H-2b,5b-pyrr); 2.86-3.00 (m, 4H, CH₂N); 3.116, 3.118 (2 × dd, 2 × 2H, *J*_{gem} = 10.6, *J*_{vic} = 5.0, H-2a,5a-pyrr); 3.42, 3.43 (2 × t, 2 × 2H, *J*_{vic} = 6.6, CH₃(CH₂)₁₃CH₂C**H₂**O); 3.515, 3.522 (2 × t, 2 × 2H, *J*_{vic} = 6.1, OCH₂CH₂C**H₂**OC₁₆H₃₃); 4.08 (m, 4H, OC**H₂**CH₂CH₂OC₁₆H₃₃); 4.11-4.21 (m, 8H, H-3',4', H-3,4-pyrr); 4.216, 4.218 (2 × dd, 2 × 1H, *J*_{2',3'} = 5.1, *J*_{2',1'} = 4.1, H-2'); 4.26 (ddd, 1H, *J*_{gem} = 11.6, *J*_{H,P} = 6.8, *J*_{5'b,4'}= 4.5, H-5'b); 4.30-4.34 (m, 2H, H-5'); 4.36 (ddd, 1H, *J*_{gem} = 11.6, *J*_{H,P} = 6.6, *J*_{5'a,4'} = 2.9, H-5'a); 5.751, 5.753 (2 × d, 2 × 1H, *J*_{5,6} = 8.1, H-5); 5.845 (d, 2H, *J*_{1',2'}= 4.1, H-1'); 7.70, 7.73 (2 × d, 2 × 1H, *J*_{6,5} = 8.1, H-6).
**¹³C NMR** (125.7 MHz, CD₃OD): 14.47 (CH₃(CH₂)₁₅); 23.73 (CH₃(CH₂)₁₄CH₂O); 24.75, 24.81 (d, *J* _{C,P} = 139.6, CH₂P); 27.28, 30.47, 30.63, 30.76, 30.79 (CH₃(CH₂)₁₄CH₂O); 31.75, 31.78 (d, *J*_{C,P} = 6.2, OCH₂CH₂CH₂OC₁₆H₃₃); 33.06 (CH₃(CH₂)₁₄CH₂O); 50.81, 50.85 (CH₂N); 60.95, 60.98 (CH₂-2,5-pyrr); 64.86, 64.93 (d, *J*_{C,P} = 6.7, OCH₂CH₂CH₂OC₁₆H₃₃); 66.52, 66.54 (d, *J*_{C,P} = 6.2, CH₂-5'); 67.49, 67.53 (OCH₂CH₂CH₂OC₁₆H₃₃); 70.79, 70.86 (CH-3'); 72.14 (CH₃(CH₂)₁₄CH₂O); 74.87, 74.93 (CH-2'); 78.12, 78.19 (CH-3,4-pyrr); 83.57, 83.58 (d, *J*_{C,P} = 6.6, CH-4'); 91.75, 91.85 (CH-1'); 103.02 (CH-5); 142.58, 142.64 (CH-6); 152.18, 152.20 (C-2); 165.99 (C-4).
**³¹P NMR** (202.3 MHz, CD₃OD): 31.37, 31.64.
**IR** vₘₐₓ(KBr) 3405 (s, vbr), 3063 (w), 2960 (m, sh), 2925 (vs), 2854 (s), 2810 (w, sh), 1696 (vs), 1629 (w, sh), 1466 (m), 1460 (m, sh), 1418 (w), 1384 (m), 1266 (m), 1223 (m), 1111 (s), 1075 (m, sh), 1046 (s, br), 998 (m), 821 (w), 765 (w), 721 (w) cm⁻¹.
**HR-ESI** C₃₄H₆₃O₁₁N₃P (M+H)⁺ calcd 720.41947; found 720.41939.

### Example 10

### Hexadecyloxypropyl cytidin-5'-yl 2-([3R,4R]-3,4-dihydroxypyrrolidin-1-N-yl)ethyl-phosphonate

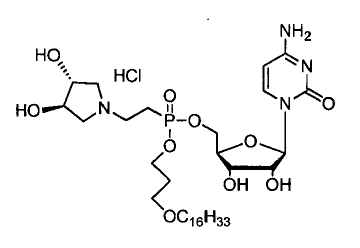

The mixture of (3R,4R)-3,4-dihydroxypyrrolidine (0.05 g, 0.49 mmol) and benzoylcytosine vinylphosphonate from example 9 (0.34 g, 0.45 mmol) in n-BuOH (10 ml) is stirred at 100 °C overnight. The solvent is removed and the protected intermediate is purified by flash chromatography on silica gel using linear gradient of H1 in ethyl acetate. White foam obtained after evaporation of solvents is without further characterization (only LC-MS confirmation) dissolved in 8M ethanolic methylamine (10 ml) and stirred at rt overnight. The mixture is concentrated *in vacuo,* co-evaporated with ethanol (2x 20 ml), dissolved in 0.2M methanolic HCl (10 ml), and the reaction mixture is stirred at rt for 4 hours. After concentration *in vacuo* the final product is obtained by preparative HPLC in 27% overall yield (90.6 mg, 120 µM) after lyofilisation from water.
A mixture of diastereoisomers ∼ 8:7
**¹H NMR** (600.1 MHz, CD₃OD): 0.90 (m, 6H, C**H**₃(CH₂)₁₅); 1.25-1.38 (m, 52H, CH₃(C**H**₂)₁₃CH₂CH₂-O); 1.55 (m, 4H, CH₃(CH₂)₁₃C**H₂**CH₂O); 1.93 (m, 4H, OCH₂C**H₂**CH₂OC₁₆H₃₃); 2.10-2.18 (m, 4H, CH₂P); 2.56 (bm, 4H, H-2b,5b-pyrr); 2.75-2.87 (bm, 4H, CH₂N); 3.00 (bm, 4H, H-2a,5a-pyrr); 3.41, 3.43 (2 × t, 2 × 2H, *J*_{vic} = 6.7, CH₃(CH₂)₁₃CH₂C**H₂**O); 3.51, 3.53 (2 × t, 2 × 2H, *J*_{vic} = 6.0, OCH₂CH₂C**H₂**OC₁₆H₃₃); 4.04 (m, 4H, H-3,4-pyrr); 4.08-4.23 (m, 10H, H-2',3',4'-pyrr, OC**H₂**CH₂CH₂OC₁₆H₃₃); 4.25-4.42 (m, 4H, H-5'); 5.83, 5.84 (d, 2H, *J*_{1',2'} = 3.0, H-1'); 5.93, 5.94 (2 × d, 2 × 1H, *J*_{5,6} = 7.6, H-5); 7.77, 7.79 (2 × d, 2 × 1H, *J*_{6,5} = 7.6, H-6).
**¹³C NMR** (150.9 MHz, CD₃OD): 14.47 (CH₃(CH₂)₁₅); 23.75 (CH₃(CH₂)₁₄CH₂O); 25.09, 25.14 (d, *J* _{C,P} = 139.1, CH₂P); 27.29, 27.30, 30.49, 30.64, 30.77, 30.80 (CH₃(CH₂)₁₄CH₂O); 31.76, 31.79 (d, *J*_{C,P} = 6.3, OCH₂CH₂CH₂OC₁₆H₃₃); 33.08 (CH₃(CH₂)₁₄CH₂O); 50.55 (CH₂N); 60.99, 61.02 (CH₂-2,5-pyrr); 64.71, 64.79 (d, *J*_{C,P} = 6.6, OCH₂CH₂CH₂OC₁₆H₃₃); 66.24, 66.31 (d, *J*_{C,P} = 6.4, CH₂-5'); 67.51, 67.55 (OCH₂CH₂CH₂OC₁₆H₃₃); 70.52, 70.55 (CH-3'); 72.15 (CH₃(CH₂)₁₄CH₂O); 75.68, 75.70 (CH-2'); 78.61, 78.66 (CH-3,4-pyrr); 83.13, 83.16 (d, *J*_{C,P} = 6.6, CH-4'); 92.89, 92.90 (CH-1'); 96.17, 96.18 (C-5); 142.56, 142.64 (CH-6); 158.26, 158.27 (C-2); 167.646, 167.653 (C-4).
**³¹P NMR** (202.3 MHz, CD₃OD): 32.67, 32.99.
**IR** *v*ₘₐₓ(CHCl₃) 3341 (m, vbr), 3220 (m, vbr), 2927 (vs), 2855 (s), 1649 (vs), 1609 (m, sh), 1578 (w), 1527 (m), 1494 (m), 1407 (m), 1468 (m), 1459 (m, sh), 1380 (m), 1287 (m), 1240 (m), 1110 (s), 1077 (m), 1046 (s), 1030 (s, sh), 996 (m) cm-¹.
**HR-ESI** C₃₄H₆₄0_{1O}N₄P (M+H)⁺ calcd 719.4355; found 719.4357

### Example 11

### Hexadecyloxypropyl uridin-5'-yl 2-([3R,5R]-3,4-dihydroxypiperidin-1-N-yl)ethyl-phosphonate

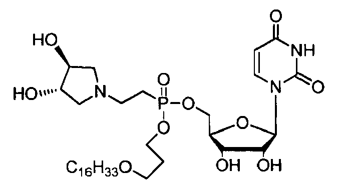

The mixture of (3S,4S)-3,4-dihydroxypyrrolidine (0.26 g, 2.56 mmol) and vinylphosphonate from example 7 (0.84 g, 1.28 mmol) in n-BuOH (30 ml) is stirred at 105 °C overnight. The solvent is removed and the isopropylidene intermediate is obtained by flash chromatography on silica gel using linear gradient of solvent mixture H1 in ethyl acetate. Obtained compound is dissolved in 0.5 M methanolic HCl (40 ml) and the mixture is stirred at rt for 4 h. The final product is obtained by chromatography on silica gel using linear gradient of H1 in ethyl acetate in 36% overall yield (344 mg, 0.46 mmol) and is lyophilized from water.
A mixture of diastereoisomers ∼ 6:5
**¹H NMR** (499.8 MHz, CD₃OD): 0.90 (m, 6H, C**H**₃(CH₂)₁₅); 1.25-1.39 (m, 52H, CH₃(C**H**₂)₁₃CH₂CH₂-O); 1.55 (m, 4H, CH₃(CH₂)₁₃C**H₂**CH₂O); 1.95 (m, 4H, OCH₂C**H₂**CH₂OC₁₆H₃₃); 2.37-2.52 (m, 4H, CH₂P); 3.16 (bm, 2H, H-2b,5b-pyrr); 3.42 (t, 2H, *J*_{vic} = 6.7, CH₃(CH₂)₁₃CH₂C**H₂**O); 3.425 (bm, 2H, H-2b,5b-pyrr); 3.43 (t, 2H, *J*_{vic} = 6.7, CH₃(CH₂)₁₃CH₂C**H₂**O); 3.515 (m, 4H, CH₂N); 3.52, 3.53 (2 × t, 2 × 2H, *J*_{vic} = 6.1, OCH₂CH₂C**H₂**OC₁₆H₃₃); 3.59, 3.92 (2 × bm, 2 × 2H, H-2a,5a-pyrr); 4.14 (m, 2H, H-4'); 4.16 (m, 2H, H-3'); 4.18-4.27 (m, 10H, H-2', OC**H₂**CH₂CH₂OC₁₆H₃₃, H-3,4-pyrr); 4.31 (ddd, 1H, *J*_{gem} = 11.5, *J*_{H,P} = 7.6, *J*_{5'b,4'} = 5.4, H-5'b); 4.36 (m, 2H, H-5'); 4.40 (ddd, 1H, *J*_{gem} = 11.5, *J*_{H,P} = 7.3, *J*_{5'a,4'} = 2.9, H-5'a); 5.742, 5.746 (2 × d, 2 × 1H, *J*_{5,6} = 8.1, H-5); 5.78, 5.80 (2 × d, 2 × 1H, *J*_{1',2'} = 3.9, H-1'); 7.68, 7.70 (2 × d, 2 × 1H, *J*_{6,5} = 8.1, H-6).
**¹³C NMR** (125.7 MHz, CD₃OD): 14.45 (CH₃(CH₂)₁₅); 23.32 (d, *J*_{C,P} = 142.0, CH₂P); 23.74 (CH₃(CH₂)₁₄CH₂O); 27.29, 30.48, 30.64, 30.76, 30.79 (CH₃(CH₂)₁₄CH₂O); 31.70, 31.72 (d, *J*_{C,P} = 6.1, OCH₂CH₂CH₂OC₁₆H₃₃); 33.07 (CH₃(CH₂)₁₄CH₂O); 52.18, 52.22 (CH₂N); 60.67, 60.75, 60.98, 61.01 (CH₂-2,5-pyrr); 65.33, 65.58 (d, *J*_{C,P} = 6.7, OCH₂CH₂CH₂OC₁₆H₃₃); 67.16 (d, *J*_{C,P} = 6.5, CH₂-5'); 67.44 (OCH₂CH₂**C**H₂OC₁₆H₃₃); 67.45 (d, *J*_{C,P} = 4.3, CH₂-5'); 67.47 (OCH₂CH₂**C**H₂OC₁₆H₃₃); 70.77 (CH-3'); 72.16 (CH₃(CH₂)₁₄**C**H₂O); 74.55, 74.60 (CH-2'); 75.67, 75.68, 76.04 (CH-3,4-pyrr); 83.29 (d, *J*_{C,P} = 6.2, CH-4'); 83.40 (d, *J*_{C,P} = 5.8, CH-4'); 92.83, 92.85 (CH-1'); 103.03, 103.10 (CH-5); 143.12, 143.19 (CH-6); 152.13, 152.14 (C-2); 166.00 (C-4).
³¹P NMR (202.3 MHz, CD₃OD): 26.79, 27.26.
**IR** vₘₐₓ(KBr) 3405 (s, vbr), 2694 (w, vbr), 2596 (w, vbr), 2925 (vs), 2854 (s), 1714 (s, sh), 1694 (vs), 1630 (m, sh), 1466 (m), 1458 (m, sh), 1418 (w), 1415 (w), 1385 (m), 1269 (m), 1229 (m, br), 1107 (s), 1053 (m, sh), 1030 (s, br), 1002 (s), 819 (w), 765 (vw), 721 (vw) cm⁻¹.
**HR-ESI** C₃₄H₆₃O₁₁N₃P (M+H)⁺ calcd 720.4195; found 720.4194

### Example 12

### Hexadecyloxypropyl uridin-5'-yl 2-([3R,4S,5S]-3,4-trihydroxypiperidin-1-N-yl)ethyl-phosphonate

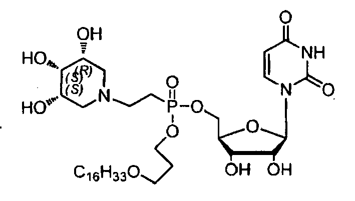

The mixture of (3R,4S,5S)-3,4,5-trihydroxypiperidine (0.04 g, 0.33 mmol) and vinylphosphonate from the example 7 (0.1 g, 0.16 mmol) in n-BuOH (5 ml) is stirred at 105 °C overnight. The solvent is removed and the isopropylidene intermediate is obtained by flash chromatography on silica gel using linear gradient of H1 in ethyl acetate. This compound is dissolved in 0.5 M methanolic HCl (20 ml) and the mixture is stirred at rt for 4 h. The final product is obtained by flash chromatography on silica gel using linear gradient of H1 in ethyl acetate in 68% overall yield (80 mg, 0.11 mmol) and is lyophilized from water.
A mixture of diastereoisomers ∼ 7:3
**¹H NMR** (499.8 MHz, CD₃OD): 0.90 (m, 6H, CH₃(C**H**₂)₁₅); 1.25-1.38 (m, 52H, CH₃(C**H**₂)₁₃CH₂CH₂-O); 1.55 (m, 4H, CH₃(CH₂)₁₃C**H₂**CH₂O); 1.93 (m, 4H, OCH₂C**H₂**CH₂OC₁₆H₃₃); 2.10-2.18 (m, 4H, CH₂P); 2.43, 2.54 (2 × bm, 2 × 4H, H-2,6-pip); 2.75 (m, 4H, CH₂N); 3.42, 3.43 (2 × t, 2 × 2H, *J*_{vic} = 6.6, CH₃(CH₂)₁₃CH₂C**H₂**O); 3.52, 3.53 (2 × t, 2 × 2H, *J*_{vic} = 6.1, OCH₂CH₂C**H₂**OC₁₆H₃₃); 3.68 (bm, 4H, H-3,5-pip); 3.81 (bm, 2H, H-4-pip); 4.11-4.22 (m, 10H, H-2',3',4', OC**H₂**CH₂CH₂OC₁₆H₃₃); 4.23-4.38 (m, 4H, H-5'); 5.746, 5.749 (2 × d, 2 × 1H, *J*_{5,6} = 8.1, H-5); 5.84 (d, 2H, *J*_{1',2'} = 4.1, H-1'); 7.71, 7.73 (2 × d, 2 × 1H, *J*_{6,5} = 8.1, H-6).
**¹³C NMR** (125.7 MHz, CD₃OD): 14.45 (CH₃(CH₂)₁₅); 23.55, 23.65 (d, *J*_{C,P} = 138.5, CH₂P); 23.75 (CH₃(CH₂)₁₄CH₂O); 27.30, 30.49, 30.63, 30.77, 30.80 (CH₃(CH₂)₁₄CH₂O); 31.79, 31.82 (d, *J*_{C,P} = 6.2, OCH₂CH₂CH₂OC₁₆H₃₃); 33.08 (CH₃(CH₂)₁₄CH₂O); 51.75 (CH₂N); 54.40 (CH₂-2,6-pip); 64.72, 64.78 (d, *J*_{C,P} = 6.5, OCH₂CH₂CH₂OC₁₆H₃₃); 66.37 (d, *J*_{C,P} = 6.3, CH₂-5'); 67.51, 67.55 (OCH₂CH₂CH₂OC₁₆H₃₃); 69.67 (CH-3,5-pip); 70.81, 70.83 (CH-3'); 71.83 (CH-4-pip); 72.17 (CH₃(CH₂)₁₄CH₂O); 74.97, 75.01 (CH-2'); 83.59, 83.63 (d, *J*_{C,P} = 6.7, CH-4'); 91.84, 91.92 (CH-1'); 103.00 (CH-5); 142.56, 142.60 (CH-6); 152.18, 152.19 (C-2); 166.05 (C-4).
**³¹P NMR** (202.3 MHz, CD₃OD): 32.91, 33.23.
**IR** vₘₐₓ(KBr) 3423 (vs, br), 2924 (s), 2854 (m), 1687 (s), 1632 (m), 1466 (m), 1406 (w), 1380 (w), 1264 (m), 1229 (m), 1116 (m), 1072 (m, sh), 1048 (m), 1029 (m), 996 (m, sh), 766 (vw) cm⁻¹. **HR-ESI** C₃₅H₆₅O₁₁N₃P (M+H)⁺ calcd 734.4347; found 734.4351

### Example 13

### Hexadecyloxypropyl uridin-5'-yl 2-([3R,5R]-3,4-dihydroxypiperidin-1-N-yl)ethylphosphonate

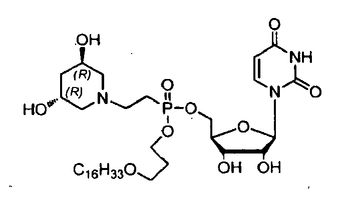

The mixture of (3R,5R)-3,5-dihydroxypiperidine (0.05 g, 0.42 mmol) and vinylphosphonate from the example 7 (0.25 g, 0.38 mmol) in n-BuOH (5 ml) is stirred at 105 °C overnight. The solvent is removed and the isopropylidene intermediate is obtained by flash chromatography on silica gel using linear gradient of H1 in ethyl acetate. Obtained compound is dissolved in 0.5 M methanolic HCl (20 ml) and the mixture is stirred at rt for 4 h. The final product is obtained by chromatography on silica gel using linear gradient of H1 in ethyl acetate in 76% overall yield (230 mg, 0.29 mmol) as a white amorphous solid.
A mixture of diastereoisomers ∼ 6:4
**¹H NMR** (600.1 MHz, CD₃OD): 0.90 (m, 6H, CH₃(C**H**₂)₁₅); 1.25-1.38 (m, 52H, CH₃(C**H**₂)₁₃CH₂CH₂-O); 1.55 (m, 4H, CH₃(CH₂)₁₃C**H₂**CH₂O); 1.70 (bm, 4H, H-4-pip); 1.93 (m, 4H, OCH₂C**H₂**CH₂OC₁₆H₃₃); 2.12-2.19 (m, 4H, CH₂P); 2.36, 2.57 (2 × m, 2 × 4H, H-2,6-pip); 2.66-2.76 (m, 4H, CH₂N); 3.42, 3.43 (2 × t, 2 × 2H, *J*_{vic} = 6.6, CH₃(CH₂)₁₃CH₂C**H₂**O); 3.51, 3.53 (2 × t, 2 × 2H, *J*_{vic} = 6.1, OCH₂CH₂C**H₂**OC₁₆H₃₃); 4.01 (m, 4H, H-3,5-pip); 4.12-4.24 (m, 10H, H-2',3',4', OC**H**₂CH₂CH₂OC₁₆H₃₃); 4.24-4.40 (m, 4H, H-5'); 5.751, 5.754 (2 × d, 2 × 1H, *J*_{5,6} = 8.1, H-5); 5.854, 5.856 (2 × d, 2 × 1H, *J*_{1',2'} =4.1, H-1'); 7.71, 7.74 (2 × d, 2 × 1H, *J*_{6,5} = 8.1, H-6).
**¹³C NMR** (150.9 MHz, CD₃OD): 14.49 (CH₃(CH₂)₁₅); 23.49, 23.53 (d, *J*_{C,P} = 138.4, CH₂P); 23.75 (CH₃(CH₂)₁₄CH₂O); 27.29, 27.30, 30.49, 30.65, 30.78, 30.81 (CH₃(CH₂)₁₄CH₂O); 31.76, 31.83 (d, *J*_{C,P} = 6.3, OCH₂CH₂CH₂OC₁₆H₃₃); 33.08 (CH₃(CH₂)₁₄CH₂O); 40.63, 40.65 (CH₂-4-pip); 52.02, 52.04 (d, *J*_{C,P} = 2.0, CH₂N); 60.07, 60.10 (CH₂-2,6-pip); 64.63, 64.75 (d, *J*_{C,P} = 6.6, OCH₂CH₂CH₂OC₁₆H₃₃); 65.58 (CH-3,5-pip); 66.27, 66.52 (d, *J*_{C,P} = 6.2, CH₂-5'); 67.51, 67.54 (OCH₂CH₂CH₂OC₁₆H₃₃); 70.77, 70.84 (CH-3'); 72.13, 72.15 (CH₃(CH₂)₁₄CH₂O); 74.99, 75.05 (CH-2'); 83.59, 83.60 (d, *J*_{C,P} = 6.6, CH-4'); 91.65, 91.66 (CH-1'); 102.99 (CH-5); 142.45, 142.51 (CH-6); 152.16, 152.18 (C-2); 165.99 (C-4).
**³¹P NMR** (202.3 MHz, CD₃OD): 33.22, 33.53.
**IR** *v*ₘₐₓ(KBr) 3431 (vs, br), 2924 (m), 2853 (w), 1710 (m, sh), 1693 (m), 1631 (m), 1468 (w), 1414 (vw), 1380 (w), 1265 (w, br), 1230 (w, br, sh), 1113 (m), 1060 (m, br), 1032 (m), 1006 (m), 764 (vw) cm⁻¹.
**HR-ESI** C₃₅H₆₅O₁₀N₃P (M+H)⁺ calcd 718.4402; found 718.4402

### Example 14

### Icosanyl uridin-5'-yl 2-([3R,4R]-3,4-dihydroxypyrrolidin-1-N-yl)ethylphosphonate

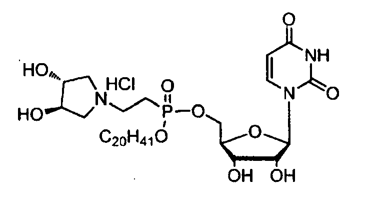

The mixture of (3R,4R)-3,4-dihydroxypyrrolidine (0.26 g, 2.52 mmol) and vinylphosphonate from the example 6 (1.1 g, 1.68 mmol) in n-BuOH (17 ml) is stirred at 100 °C overnight. The solvent is removed and the isopropylidene intermediate is obtained by flash chromatography on silica gel using linear gradient of H1 in ethyl acetate. This compound is dissolved in 0.5 M methanolic HCl (50 ml), and the mixture is stirred at rt for 4 h. The final product is obtained by flash chromatography on silica gel using linear gradient of H1 in ethyl acetate in 70% overall yield (840 mg, 1.17 mmol) and lyophilized from water.
A mixture of two diastereoisomers ∼ 6:5
**¹H NMR** (600.1 MHz, CD₃OD): 0.90 (m, 6H, C**H**₃(CH₂)₁₉); 1.25-1.43 (m, 68H, CH₃(C**H**₂)₁₇CH₂CH₂-O); 1.69 (m, 4H, CH₃(CH₂)₁₇C**H**₂CH₂O); 2.06-2.23 (m, 4H, CH₂P); 2.68 (dd, 4H, *J*_{gem} = 10.6, *J*_{vic} = 2.9, H-2b,5b-pyrr); 2.84-2.97 (m, 4H, CH₂N); 3.10 (dd, 4H, *J*_{gem} = 10.6, *J*_{vic} = 5.0, H-2a,5a-pyrr); 4.07 (m, 4H, H-3,4-pyrr); 4.08-4.13 (m, 4H, CH₃(CH₂)₁₇CH₂C**H**₂O); 4.13-4.16 (m, 4H, H-3',4'); 4.207, 4.210 (2 × dd, 2 × 1H, *J*_{2',3'} = 5.0, *J*_{2',1'} = 4.2, H-2'); 4.25 (ddd, 1H, *J*_{gem} = 11.6, *J*_{H,P} = 6.8, *J*_{5'b,4'} = 4.4, H-5'b); 4.31 (m, 2H, H-5'); 4.35 (ddd, 1H, *J*_{gem} = 11.6, *J*_{H,P} = 6.6, *J*_{5'a,4'} = 2.7, H-5'a); 5.735, 5.743 (2 × d, 2 × 1H, *J*_{5,6} = 8.1, H-5); 5.84, 5.85 (2 × d, 2 × 1H, *J*_{1',2'} = 4.2, H-1'); 7.71, 7.74 (2 × d, 2 × 1H, *J*_{6,5} = 8.1, H-6).
¹³C NMR (150.9 MHz, CD₃OD): 14.46 (CH₃(CH₂)₁₉); 23.75 (CH₃(CH₂)₁₇CH₂CH₂O); 24.85, 24.91 (d, *J*_{C,P} = 139.8, CH₂P); 26.64, 30.28, 30.29, 30.49, 30.69, 30.73, 30.74, 30.77, 30.79, 30.80 (CH₃(CH₂)₁₇CH₂CH₂O); 31.55, 31.57 (d, *J*_{C,P} = 6.0, CH₃(CH₂)₁₇CH₂CH₂O); 33.09 (CH₃(CH₂)₁₇CH₂CH₂O); 50.81, 50.86 (CH₂N); 60.98, 61.02 (CH₂-2,5-pyrr); 66.52, 66.54 (d, *J*_{C,P} = 6.1, CH₂-5'); 67.69, 67.73 (d, *J*_{C,P} = 7.1, CH₃(CH₂)₁₇CH₂CH₂O); 70.80, 70.87 (CH-3'); 74.90, 74.95 (CH-2'); 78.19, 78.26 (CH-3,4-pyrr); 83.60 (d, *J*_{C,P} = 6.5, CH-4'); 91.75, 91.93 (CH-1'); 102.97, 102.99 (CH-5); 142.65 (CH-6); 152.17, 152.20 (C-2); 166.03 (C-4).
**³¹P NMR** (202.3 MHz, CD₃OD): 31.29, 31.57.
**IR** *v*ₘₐₓ(KBr) 3354 (m, vbr), 3063 (m), 2927 (vs), 2855 (s), 1694 (vs), 1630 (w, sh), 1467 (m), 1457 (m, sh), 1388 (m), 1267 (m), 1240 (m, sh), 1108 (m), 1075 (m), 1037 (s, br), 999 (s) cm⁻¹.
**HR-ESI** C₃₅H₆₅O₁₀N₃P (M+H)⁺ calcd 718.44021, found 718.44030.

### Example 15

### Octadecyl uridin-5'-yl 2-([3R,4R]-3,4-dihydroxypyrrolidin-1-N-yl)ethylphosphonate

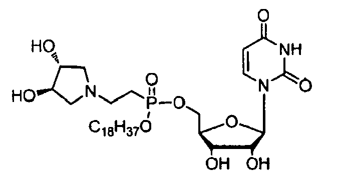

The mixture of (3R,4R)-3,4-dihydroxypyrrolidine (0.3 g, 2.94 mmol) and vinylphosphonate from the example 5 (1.23 g, 1.96 mmol) in n-BuOH (20 ml) is stirred at 100 °C overnight. The solvent is removed and the isopropylidene intermediate is obtained by flash chromatography on silica gel using linear gradient of H1 in ethyl acetate. Obtained compound is dissolved in 0.5 M methanolic HCl (50 ml), and the mixture is stirred at rt for 4 h. The final product is obtained by flash chromatography on silica gel using linear gradient of H1 in ethyl acetate in 68% overall yield (920 mg, 1.33 mmol) and lyophilized from water.
A mixture of two diastereoisomers ∼ 6:5
**¹H NMR** (600.1 MHz, CD₃OD): 0.90 (m, 6H, CH₃(C**H**₂)₁₇); 1.25-1.43 (m, 60H, CH₃(C**H**₂)₁₅CH₂CH₂-O); 1.68 (m, 4H, CH₃(CH₂)₁₅C**H**₂CH₂O); 2.06-2.19 (m, 4H, CH₂P); 2.55 (m, 4H, H-2b,5b-pyrr); 2.73-2.86 (m, 4H, CH₂N); 2.99 (m, 4H, H-2a,5a-pyrr); 4.04 (m, 4H, H-3,4-pyrr); 4.05-4.13 (m, 4H, CH₃(CH₂)₁₅CH₂C**H**₂O); 4.13-4.16 (m, 4H, H-3',4'); 4.199, 4.201 (2 × dd, 2 × 1H, *J*_{2',3'}= 4.8, *J*_{2',1'} = 4.2, H-2'); 4.24 (ddd, 1H, *J*_{gem} = 11.6, *J*_{H,P} = 6.7, *J*_{5'b,4'} = 4.2, H-5'b); 4.28 (ddd, 1H, *J*_{gem} = 11.6, *J*_{H,P} = 7.4, *J*_{5'b,4'}= 2.5, H-5'b); 4.30 (ddd, 1H, *J*_{gem} = 11.6, *J*_{H,P} = 5.2, *J*_{5'a,4'} = 2.7, H-5'a); 4.34 (ddd, 1H, *J*_{gem} = 11.6, *J*_{H,P} = 6.5, *J_{5'a,4'}* = 2.7, H-5'a); 5.735, 5.745 (2 × d, 2 × 1H, *J*_{5,6} = 8.1, H-5); 5.85, 5.86 (2 × d, 2 × 1H, *J*_{1',2'} = 4.2, H-1'); 7.71, 7.74 (2 × d, 2 × 1H, *J*_{6,5} = 8.1, H-6).
**¹³C NMR** (150.9 MHz, CD₃OD): 14.47 (CH₃(CH₂)₁₇); 23.75 (CH₃(CH₂)₁₅CH₂CH₂O); 25.13, 25.17 (d, *J*_{C,P} = 139.3, CH₂P); 26.65, 30.28, 30.29, 30.49, 30.68, 30.69, 30.72, 30.73, 30.78, 30.79, 30.80 (CH₃(CH₂)₁₅CH₂CH₂O); 31.55, 31.57 (d, *J*_{C,P} = 6.0, CH₃(CH₂)₁₅CH₂CH₂O); 33.09 (CH₃(CH₂)₁₅CH₂CH₂O); 50.55, 50.57 (d, *J*_{C,P} = 1.1, CH₂N); 61.01, 61.04 (CH₂-2,5-pyrr); 66.34, 66.42 (d, *J*_{C,P} = 6.3, CH₂-5'); 66.60, 67.62 (d, *J*_{C,P} = 6.8, CH₃(CH₂)₁₅CH₂CH₂O); 70.80, 70.88 (CH-3'); 74.96, 75.02 (CH-2'); 78.63, 78.68 (CH-3,4-pyrr); 83.63, 83.65 (d, *J*_{C,P} = 6.7, CH-4'); 91.56, 91.74 (CH-1'); 102.95, 102.98 (CH-5); 142.55, 142.57 (CH-6); 152.18, 152.22 (C-2); 166.03 (C-4).
**³¹P NMR** (202.3 MHz, CD₃OD): 32.12, 32.43.
**IR** *v*ₘₐₓ(CHCl₃) 3374 (m, vbr), 3063 (m), 2927 (vs), 2855 (s), 1694 (vs), 1630 (m, sh), 1467 (s), 1457 (s, sh), 1388 (m), 1267 (s), 1239 (m, sh), 1109 (s), 1074 (s), 1040 (s, br), 997 (s) cm⁻¹.
**HR-ESI** C₃₃H₆₁O₁₀N₃P (M+H)⁺ calcd 690.40891, found 690.40891.

### Example 16

### Hexadecyl uridin-5'-yl 2-([3R,4R]-3,4-dihydroxypyrrolidin-1-N-yl)ethylphosphonate

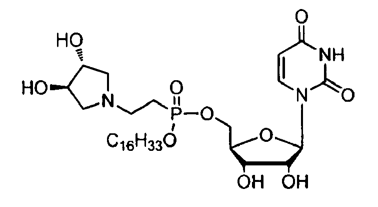

The mixture of (3R,4R)-3,4-dihydroxypyrrolidine (0.36 g, 3.45 mmol) and vinylphosphonate from the example 4 (1.38 g, 2.3 mmol) in n-BuOH (20 ml) is stirred at 100 °C overnight. The solvent is removed and isopropylidene intermediate is obtained by flash chromatography on silica gel using linear gradient of H1 in ethyl acetate. Obtained compound is dissolved in 0.5 M methanolic HCl (50 ml), and the mixture is stirred at rt for 4 h. The final product is obtained by flash chromatography on silica gel using linear gradient of H1 in ethyl acetate in 38% overall yield (566 mg, 0.86 mmol) and is lyophilized from water.
A mixture of two diastereoisomers ∼ 1:1
**¹H NMR** (600.1 MHz, CD₃OD): 0.90 (m, 6H, C*H*₃(CH₂)₁₅); 1.24-1.43 (m, 52H, CH₃(C**H**₂)₁₃CH₂CH₂-O); 1.68 (m, 4H, CH₃(CH₂)₁₃C**H**₂CH₂O); 2.06-2.18 (m, 4H, CH₂P); 2.55 (m, 4H, H-2b,5b-pyrr); 2.73-2.86 (m, 4H, CH₂N); 2.99 (m, 4H, H-2a,5a-pyrr); 4.04 (m, 4H, H-3,4-pyrr); 4.05-4.12 (m, 4H, CH₃(CH₂)₁₃CH₂C**H**₂O); 4.12-4.16 (m, 4H, H-3',4'); 4.198, 4.200 (2 × dd, 2 × 1H, *J*_{2',3'} = 5.1, *J*_{2',1'} = 4.1, H-2'); 4.24 (ddd, 1H, *J*_{gem} = 11.6, *J*_{H,P} = 6.5, *J*_{5'b,4'} = 4.2, H-5'b); 4.28 (ddd, 1H, *J*_{gem} = 11.6, *J*_{H,P} = 7.2, *J*_{5'b,4'} = 2.4, H-5'b); 4.30 (ddd, 1H, *J*_{gem} = 11.6, *J*_{H,P} = 5.2, *J*_{5'a,4'} = 2.7, H-5'a); 4.34 (ddd, 1H, *J*_{gem} = 11.6, *J*_{H,P} = 6.6, *J*_{5'a,4'} = 2.8, H-5'a); 5.73, 5.74 (2 × d, 2 × 1H, *J*_{5,6} = 8.1, H-5); 5.85, 5.86 (2 × d, 2 × 1H, *J*_{1',2'} = 4.1, H-1'); 7.71, 7.74 (2 × d, 2 × 1H, *J*_{6,5} = 8.1, H-6).
**¹³C NMR** (150.9 MHz, CD₃OD): 14.46 (CH₃(CH₂)₁₅); 23.75 (CH₃(CH₂)₁₃CH₂CH₂O); 25.14, 25.19 (d, *J*_{C,P} = 139.3, CH₂P); 26.65, 30.27, 30.28, 30.50, 30.68, 30.69, 30.72, 30.73, 30.78, 30.81 (CH₃(CH₂)₁₃CH₂CH₂O); 31.55, 31.57 (d, *J*_{C,P} = 6.0, CH₃(CH₂)₁₃CH₂CH₂O); 33.09 (CH₃(CH₂)₁₃CH₂CH₂O); 50.55, 50.59 (d, *J*_{C,P} = 1.0, CH₂N); 61.02, 61.05 (CH₂-2,5-pyrr); 66.34, 66.42 (d, *J*_{C,P} = 6.3, CH₂-5'); 66.60, 67.63 (d, *J*_{C,P} = 6.8, CH₃(CH₂)₁₃CH₂CH₂O); 70.81, 70.89 (CH-3'); 74.96, 75.02 (CH-2'); 78.65, 78.69 (CH-3,4-pyrr); 83.64, 83.66 (d, *J*_{C,P} = 6.6, CH-4'); 91.58, 91.76 (CH-1'); 102.94, 102.98 (CH-5); 142.57, 142.58 (CH-6); 152.18, 152.22 (C-2); 166.04 (C-4).
³¹P NMR (202.3 MHz, CD₃OD): 32.33, 32.63.
IR *v*ₘₐₓ(CHCl₃) 3376 (m, br), 3062 (w), 2927 (vs), 2855 (s), 1693 (vs), 1632 (w, sh), 1466 (m), 1459 (m, sh), 1388 (m), 1267 (s), 1237 (m, sh), 1109 (s), 1074 (s), 1039 (s, br), 997 (s) cm⁻¹.
HR-ESI C₃₁H₅₇O₁₀N₃P (M+H)⁺ calcd 662.37761, found 662.37759.

### Example 17

### Tetradecyl uridin-5'-yl 2-([3R,4R]-3,4-dihydroxypyrrolidin-l-N-yl)ethylphosphonate

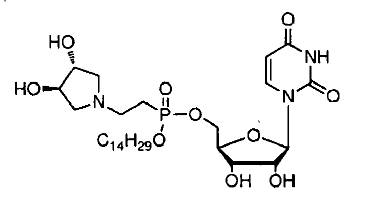

The mixture of (3R,4R)-3,4-dihydroxypyrrolidine (0.42 g, 4.10 mmol) and vinylphosphonate from the example 2 (1.17 g, 2.05 mmol) in n-BuOH (20 ml) is stirred at 100 °C overnight. The solvent is removed and the isopropylidene intermediate is obtained by flash chromatography on silica gel using linear gradient of H1 in ethyl acetate. This compound is dissolved in 0.5 M methanolic HCl (50 ml), and the mixture is stirred at rt for 4 h. The final product is obtained by flash chromatography on silica gel using linear gradient of H1 in ethyl acetate in 66% overall yield (862 mg, 1.36 mmol) and is lyophilized from water.
A mixture of two diastereoisomers ∼ 6:5
**¹H NMR** (600.1 MHz, CD₃OD): 0.90 (m, 6H, C**H**₃(CH₂)₁₃); 1.25-1.43 (m, 44H, CH₃(C**H**₂)₁₁CH₂CH₂-O); 1.68 (m, 4H, CH₃(CH₂)₁₁C**H**₂CH₂O); 2.06-2.19 (m, 4H, CH₂P); 2.56 (m, 4H, H-2b,5b-pyrr); 2.74-2.88 (m, 4H, CH₂N); 3.00 (m, 4H, H-2a,5a-pyrr); 4.04 (m, 4H, H-3,4-pyrr); 4.06-4.13 (m, 4H, CH₃(CH₂)₁₁CH₂C**H**₂O); 4.13-4.16 (m, 4H, H-3',4'); 4.200, 4.202 (2 × dd, 2 × 1H, *J*_{2',3'} = 5.0, *J*_{2',1'} = 4.0, H-2'); 4.24 (ddd, 1H, *J*_{gem} = 11.6, *J*_{H,P} = 6.7, *J*_{5'b,4'} = 4.3, H-5'b); 4.29 (ddd, 1H, *J*_{gem} = 11.6, *J*_{H,P} = 7.2, *J*_{5'b,4'} = 2.4, H-5'b); 4.31 (ddd, 1H, *J*_{gem} = 11.6, *J*_{H,P} = 5.0, *J*_{5'a,4'} = 2.6, H-5'a); 4.35 (ddd, 1H, *J*_{gem} = 11.6, *J*_{H,P} = 6.6, *J*_{5'a,4'} = 2.8, H-5'a); 5.736, 5.745 (2 × d, 2 × 1H, *J*_{5,6} = 8.1, H-5); 5.85, 5.86 (2 × d, 2 × 1H, *J*_{1',2'} = 4.0, H-1'); 7.71, 7.74 (2 × d, 2 × 1H, *J*_{6,5} = 8.1, H-6).
**¹³C NMR** (150.9 MHz, CD₃OD): 14.46 (CH₃(CH₂)₁₃); 23.75 (CH₃(CH_{Z})₁₁CH₂CH₂O); 25.11 (d, *J*_{C,P} = 139.5, CH₂P); 25.16 (d, *J*_{C,P} = 139.0, CH₂P); 26.65, 30.27, 30.28, 30.50, 30.68, 30.71, 30.72, 30.78, 30.80, 30.82 (CH₃(CH₂)₁₁CH₂CH₂O); 31.55, 31.57 (d, *J*_{C,P} = 6.0, CH₃(CH₂)₁₁CH₂CH₂O); 33.09 (CH₃(CH₂)₁₁CH₂CH₂O); 50.57, 50.60 (d, *J*_{C,P} = 1.0, CH₂N); 61.01, 61.03 (CH₂-2,5-pyrr); 66.36, 66.42 (d, *J*_{C,P} = 6.3, CH₂-5'); 66.60, 67.63 (d, *J*_{C,P} = 6.8, CH₃(CH₂)₁₁CH₂CH₂O); 70.81, 70.88 (CH-3'); 74.95, 75.01 (CH-2'); 78.60, 78.65 (CH-3,4-pyrr); 83.63, 83.64 (d, *J*_{C,P} = 6.6, CH-4'); 91.57, 91.75 (CH-1'); 102.95, 102.98 (CH-5); 142.56, 142.57 (CH-6); 152.18, 152.22 (C-2); 166.03 (C-4). ³¹P NMR (202.3 MHz, CD₃OD): 32.08, 32.38.
**IR** *v*ₘₐₓ(CHCl₃) 3374 (m, vbr), 3063 (w), 2927 (vs), 2855 (s), 1694 (vs), 1630 (w, sh), 1467 (m), 1459 (m, sh), 1388 (m), 1267 (m), 1238 (m, sh), 1109 (m), 1074 (m), 1040 (s, br), 997 (s) cm⁻¹.
**HR-ESI** C₂₉H₅₃O₁₀N₃P (M+H)⁺ calcd 634.34631, found 634.34635.

### B) Antibacterial activity

Antimicrobial activity is assessed using the standard microdilution method determining the minimum inhibitory concentration (MIC) of the tested samples leading to inhibition of bacterial growth. Disposable microtitration plates are used for the tests. The samples were diluted in brain heart infusion broth (Himedia, Laboraties Pvt. Ltd., 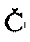 eská republika) and Mueller-Hinton broth (HiMedia Laboraties, see above) to yield a concentration range between 200 µg/ml and 1.5625 µg/ml. The plates are inoculated with a standard amount of the tested microbe - the inoculum density in each well is equal to 10⁵⁻⁶ CFU/ml. The MIC is read after 24/48 hours of incubation at 37°C as the minimum inhibitory concentration of the tested substance that inhibits the growth of the bacterial strains. The minimum bactericidal concentration (MBC) is characterized as the minimum concentration of the sample required to achieve irreversible inhibition, i.e. killing the bacterium after a defined period of incubation. The MBC is examined by the inoculation method. With an applicator, 10 µL are transferred from microplate wells with defined concentrations of the tested sample and inoculated onto the surface of blood agar (Trios, Czech Republic). The MBC is determined as the lowest concentration that inhibited the visible growth of the used bacterium.

Standard reference bacterial strains *(E. faecalis* CCM 4224, *S. aureus* CCM 4223) from the Czech Collection of Microorganisms (CCM), Faculty of Science, Masaryk University Brno, *S. agalactiae, B. subtilis,* methicilin-resistant *S. aureus* 4591, fluoroquinolone-resistant *S. haemolyticus* 16568, vancomycin-resistant *E. faecium* VanA419/ana and methicilin-resistant *S. epidermidis* 8700/B strains obtained from the Teaching Hospital Olomouc were used. All tested microorganisms were stored in cryotubes (ITEST plus, Czech Republic) at -80°C.

**Table 1**

| | | | | |
|---|---|---|---|---|
| Minimum inhibitory concentration of selected novel lipophosphonoxines against a panel of reference bacterial strains | | | | |

| | MIC µg/ml | | | |
|---|---|---|---|---|
| Compound prepared in example | *E. faecalis* CCM 4224 | *S.aureus* CCM 4223 | *B. subtilis* | *S.agalactiae* |
| **9** | 12,5 | - | 6,25 | 3,125 |
| **10** | 6,25 | 25 | 3,125 | 3,125 |
| **11** | 12,5 | - | 6,25 | 6,25 |
| **12** | - | - | - | 12,5 |
| **13** | - | - | - | 6,25 |
| **14** | - | - | - | 6,25 |
| **15** | 6,25 | 25-12,5 | 12,5-6,25 | 3,125 |
| **16** | 6.25 | 12,5-6,25 | 3,125 | 1,5625 |
| **17** | 12,5 | 25-12.5 | 6,25 | 6,25 |

**Table 2**

| | | | | |
|---|---|---|---|---|
| Minimum inhibitory concentration of selected novel lipophosphonoxines against a panel of resistant bacterial strains | | | | |

| | MIC µg/ml | | | |
|---|---|---|---|---|
| Compound prepared in example | *S. aureus* MRSA 4591 | *S. haemolyticus* 16568 | *E. faecium* VanA 419/ana | *S. epidermidis* 8700/B |
| **9** | - | - | 6,25 | 3,125 |
| **10** | - | - | 6,25 | 6,25 |
| **11** | - | - | 6,25 | 6,25 |
| **15** | - | - | 6,25 | 6,25 |
| **16** | 12,5 | 25-12,5 | 3,125 | 3,125 |
| **17** | 25 | 12,5 | 12,5 | 12,5 |

Multiresistant bacterial strains isolated from clinical isolates of patients from Teaching Hospital Olomouc: MRSA - (methicilin-resistant *S. aureus* 4591); *S. haemolyticus* (fluoroquinolone-resistant strain 16568); *E. faecium* (vancomycin-resistant strain VanA419/ana); *S. epidermidis* (methicilin-resistant strain 8700/B).

In all cases the minimum inhibitory concentration (MIC, the concentration of tested compound at which the growth of 100 % of the tested bacteria was inhibited) is equal to minimum bactericidal concentration (MBC, the concentration of tested compound at which 100% of the tested bacteria is killed). The value of MBC is determined by revaccination of bacteria tested for MIC into an inhibitor free medium and monitoring the potential growth.

### Methods of determination of cell viability and cytotoxicity:

Human immature erythroid progenitors isolated from umbilical cord blood are cultivated in the StemSpan serum-free medium (StemCell Technologies) in the presence of the stem cell factor (SCF, 100 ng/ml), erythropoetin (EPO, 33.34 nkat/ml) and dexamethasone (1 µmol.l⁻¹) (Panzenbock et al. 1998). Cells are plated at 25000 cells/well/20 µl in 384-well plates immediately before addition of lipophosphonoxine compound from particular examples. After 48 hours of incubation at 37 °C in 5% CO₂ viability is determined by using the commercial CellTiter-Blue® Cell Viability Assay (Promega, Cat.# G8082). The assay is based on the ability of living cells to convert a redox dye (resazurin) into a fluorescent end product (resorufin). Non-viable cells rapidly lose metabolic capacity and thus do not generate a fluorescent signal. The homogeneous assay procedure involves adding the single reagent directly to cells cultured in serum-supplemented medium. After an incubation step, data are recorded.

Cytotoxicity is determined after 4 hours at 37 °C in 5% CO₂ using commercial CytoTox-ONE™ Homogeneous Membrane Integrity Assay (Promega, Cat.# G7892), a fluorometric method for estimating the number of non-viable cells present in multiwell plates. The CytoTox-ONE™ Assay measures the release of lactate dehydrogenase (LDH) from the cells with a damaged membrane. LDH released into the culture medium is measured with a 10-minute coupled enzymatic assay that results in the conversion of resazurin into a fluorescent resorufin product. The amount of fluorescence produced is proportional to the number of lysed cells.

The fluorescence intensity is measured using the EnVision plate reader (PerkinElmer). Data are analyzed using GraphPad Prism 5.0 statistical software.

**Table 3**

| | | |
|---|---|---|
| Maximum safety concentration of selected novel lipophosphonoxins based on viability of normal erythroid progenitor cells using commercial test CellTiter Blue (Promega Corp., Madison, USA): | | |

| Compound prepared in example | [Max. safety concentration] µg/ml | IC₅₀ µg/ml |
|---|---|---|
| **9** | 62 | 93 |
| **10** | 31 | 46 |
| **11** | 31 | 40 |
| **12** | 62 | 141 |
| **13** | 15 | 45 |
| **14** | 15 | 29 |
| **15** | 31 | 38 |
| **16** | 31 | 44 |
| **17** | 31 | 47 |

**Table 4**

| | | |
|---|---|---|
| Cytotoxicity of selected newly prepared lipophosphonoxins: | | |

| Compound prepard in example | [Max. safety concentration] µg/ml | EC₅₀ µg/ml |
|---|---|---|
| **9** | 125 | 436 |
| **14** | 125 | 185 |
| **15** | 62 | 170 |
| **16** | 62 | 237 |
| **17** | 125 | 302 |

Cytotoxicity of tested compounds against normal human erythroid progenitor cells was determined using commercial test CytoToxOne (Promega Corp., Madison, USA)

### Industrial Applicability

Lipophosphonoxins according to this invention can be used as antibacterial agents in pharmaceutical preparations destined for the treatment of bacterial infections, components of disinfectants and/or selective growth media.

## Claims

1. Lipophosphonoxins of general formula I, 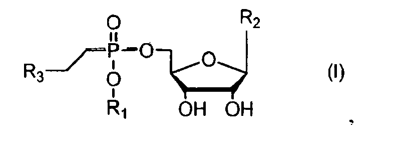 wherein R₁ is selected from a group comprising (C₈-C₂₂)alkyl, hexadecyloxypropyl, tetradecyloxypropyl, tetradecyloxyethyl, hexadecyloxyethyl,
R₂ is selected from a group comprising uracil, thymin, cytosin,
R₃ is selected from the group comprising compounds of general formula II and III: 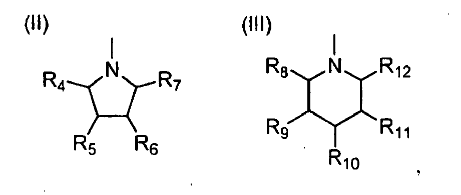 wherein R₄ is H or CH₂OH, R₅ is H or OH, R₆ is H or OH, R₇ is H or CH₂OH, R₈ is H or CH₂OH, R₉ is H or OH, R₁₀ is H or OH, R₁₁ is H or OH, R₁₂ is H or CH₂OH,
diastereomers and mixtures of diaslereomers thereof, and
their pharmaceutically acceptable salts and hydrates.

2. A method of preparation of lipophosphonoxins of general formula I according to claim 1 or diastereomers and mixtures od diastereomers thereof, **characterized in that** it contains steps A-D, in which
A) dimethyl vinylphosphonate is heated overnight with 40% to 70% (v/v) aqueous pyridine at a temperature from 50 to 80 °C affording methyl vinylphosphonate **IX** 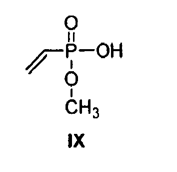
B) the obtained methyl vinylphosphonate is esterified with nucleoside **X,** protected in positions 2'and 3' with a protecting group selected from 2'.3'-isopropylidene, 2',3'-bis(diniethoxytrityl), 2',3'-dibenzoyl, or 2',3'-diacetyl, and possessing free 5'-hydroxyl group 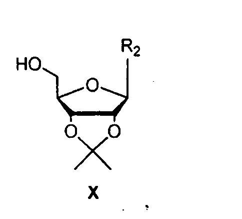 in the presence of triisopropylbenzenesulfonylchloride and under catalysis by *N*-methylimidazole in a solvent selected from the group comprising acetonitrile, dioxane, tetrahydrofuran, dichloroethane, chloroform and dichloromethane; the obtained methylester is isolated by chromatography on silica gel using linear gradient of ethanol in chloroform, and subsequently, the methyl ester group is removed by heating overnight with 40% to 70% (v/v) aqueous pyridine at a temperature from 50 to 80 °C affording vinylphosphonic acid **XI** 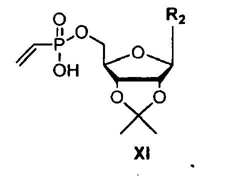 which is isolated by chromatography on silica gel using linear gradient of mixture of ethyl acetate:acetone:ethanol:water in volume ratios 4:1:1:1, in ethyl acetate;
C) the vinylphosphonic acid **XI** is esterified by alcohol using triisopropylbenzenesulfonylchloride as condensing agent and under catalysis by *N*-methylimidazole in a solvent selected from the group comprising acetonitrile, dioxane, tetrahydrofuran, dichloroethane, chloroform and dichloromethane, and the thereby obtained vinylphosphonate **XII** 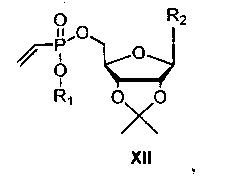 is separated by chromatography on silica gel using linear gradient of ethanol in chloroform;
D) Michael addition of secondary amine to the vinyl function of the vinylphosphonate **XII** is performed by heating in an organic solvent selected from the group comprising acetonitrile, dioxane, tetrahydrofuran, dichloroechane, chloroforme, etanol, propanol, isopropanol a n-butanol at temperature from 80 to 120 °C for 4 to 12 hours, followed by removal of the protecting groups on nucleoside; the product is subsequently isolated by chromatography on silica gel using linear gradient of mixture of ethyl acetate:acetone:ethanol:water in volume ratios 4:1:1:1, in ethyl acetate; or it is re-purified by reverse phase high performance liquid chromatography.

3. The method of preparation according to claim 2, **characterized in that** heating overnight with aqueous pyridine in step A) and/or B) is carried out at 60 °C and the concentration of the pyridine solution is 60% (v/v).

4. The method of preparation according to claim 2, **characterized in that** Michael addition in the step D is carried out in n-butanol at 105 °C. preferably overnight.

5. The method of preparation according to claim 2, **characterized in that** esterification in the steps B and/or C is preferably carried out in dichloromethane.

6. The method of preparation according to claim 2, **characterized in that** in the step D), the nucleoside protecting groups selected from 2',3'-bis(dimethoxytrityl), 2',3'-dibenzoyl and 2',3'-diacetyl are removed by ethanolic methylamine or aqueous ammonia.

7. The method of preparation according to claim 2, **characterized in that** in the step D), the 2',3'-isopropylidene protecting group is removed by treatment with 0,5 mol.1⁻¹ hydrochloride in methanol at room temperature overnight.

8. The lipophoshonoxins of general formula I or their diastereomers or pharmaceutically acceptable salts and hydrates thereof and/or mixtures of such compounds according to claim 1 for use as medicaments.

9. Lipophoshonoxins of general formula I or their diastereomers or pharmaceutically acceptable salts and hydrates thereof and/or mixtures of such compounds according to claim 1 for use as antibacterial medicaments.

10. Lipophoshonoxins of general formula I or their diastereomers or pharmaceutically acceptable salts and hydrates thereof and/or mixtures of such compounds according to claim 1 for use as an active component of disinfectants and/or selective growth media for *in vitro* cultivations.

11. Antibacterial medicament, disinfectant and/or selective growth medium containing as an active ingredient at least one lipophoshonoxin of general formula I or their diastereomers and mixtures of such diastereomers or pharmaceutically acceptable salts and hydrates thereof and/or mixtures of such compounds according to claim 1.

## Patentansprüche

1. Lipophosphonoxine der allgemeinen Formel I, 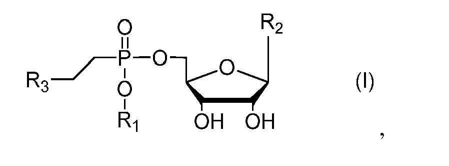 worin der Substituent R₁ aus einer Gruppe umfassend (C₈-C₂₂)Alkyl, Hexadecyloxypropyl, Tetradecyloxypropyl, Tetradecyloxyethyl, Hexadecyloxyethyl ausgewählt ist,
R₂ aus einer Gruppe umfassend Uracil, Thymin, Cytosin ausgewählt ist,
R₃ aus einer Gruppe ausgewählt ist, welche die Verbindungen der allgemeinen Formeln II und III umfasst: 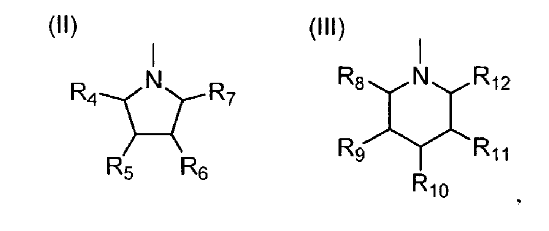 worin R₄ H oder CH₂OH ist, R₅ H oder OH ist, R₆ H oder OH ist, R₇ H oder CH₂OH ist, R₈ H oder CH₂OH ist, R₉ H oder OH ist, R₁₀ H oder OH ist, R₁₁ H oder OH ist, R₁₂ H oder CH₂OH ist,
deren Diastereomere und Diastereomer-Gemische, und
deren pharmazeutisch annehmbare Salze und Hydrate.

2. Verfahren zur Herstellung von Lipophosphonoxinen der allgemeinen Formel I nach dem Anspruch 1 oder deren Diastereomeren und Gemischen deren Diastereomere, **dadurch gekennzeichnet, dass** es die Schritte A-D umfasst, wobei
A) Dimethylvinylphosphonat über Nacht mit 40% bis 70% (Vol.-%) wässriges Pyridin bei einer Temperatur von 50 bis 80°C unter Entstehung von Methylvinylphosphonat **IX** erwärmt wird 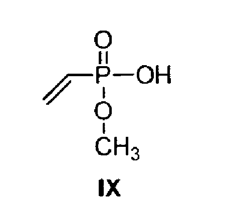
B) gewonnenes Methylvinylphosphonat esterifiziert mit dem Nukleosid **X**, geschützt in den Lagen 2' und 3' durch eine Schutzgruppe, ausgewählt aus 2',3'-Isopropyliden, 2',3'-Bis(Dimethoxytrityl), 2',3'-Dibenzoyl oder 2',3'-Diacetyl, besitzend eine freie 5'-HydroxylGruppe 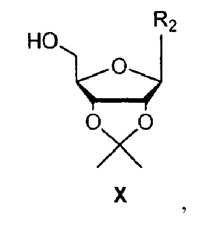 in Anwesenheit von Triisopropylbenzensulfonylchlorid und unter Katalyse durch N-Methylimidazol im Lösungsmittel, ausgewäht aus einer Gruppe, umfassend Acetonitril, Dioxan, Tetrahydrofuran, Dichlorethan, Chloroform und Dichlormethan; gewonnenes Methylester wird vermittels Chromatographie auf Silikagel unter Verwendung von linearem Ethanolgradient im Chloroform isoliert, und nachfolgend wird die Methylester-Gruppe durch das Erwärmen über Nacht mit 40% bis 70% (Vol.-%) wässriges Pyridin bei einer Temperatur von 50 bis 80°C unter Entstehung von Vinylphosphonsäure **XI** beseitigt 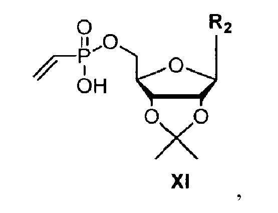 die vermittels Chromatographie auf Silikagel unter Verwendung von linearem Gradient des Gemisches von Ethylacetat:Aceton:Ethanol:Wasser im Vol.-Verhältnis 4:1:1:1, im Ethylacetat isoliert wird;
C) die Vinylphosphonsäure **XI** wird esterifiziert mit dem Alkohol mit Hilfe von Triisopropylbenzensulfonylchlorid als Kondensationsmittel und unter Katalyse durch N-Methylimidazol im Lösungsmittel, ausgewählt aus einer Gruppe, umfassend Acetonitril, Dioxan, Tetrahydrofuran, Dichlorethan, Chloroform und Dichlormethan, und das entstandene Vinylphosphonat **XII** 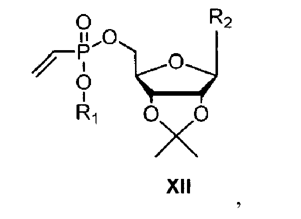 wird vermittels Chromatographie auf Silikagel unter Verwendung von linearem Ethanolgradient im Chloroform abgetrennt;
D) Michael-Addition von Sekundäramin auf die Vinylfunktion des Vinylphosphonats **XII** durch das Erwärmen wird durchgeführt im organischen Lösungsmittel, ausgewählt aus einer Gruppe, umfassend Acetonitril, Dioxan, Tetrahydrofuran, Dichlorethan, Chloroform, Ethanol, Propanol, Isopropanol und n-Butanol, bei einer Temperatur 80 bis 120 °C für 4 bis 12 Stunden, gefolgt durch die Beseitigung der Schutzgruppen auf Nukleosid; das Produkt wird weiter vermittels Chromatographie auf Silikagel unter Verwendung von linearem Gradient des Gemisches Ethylacetat:Aceton:Ethanol:Wasser im Vol.-Verhältnis 4:1:1:1 im Ethylacetat isoliert; oder es wird mit Hilfe einer hocheffektiven Chromatographie auf der Reverse-Phase nachgereinigt.

3. Verfahren zur Herstellung nach dem Anspruch 2, **dadurch gekennzeichnet, dass** das Erwärmen über Nacht mit wässrigem Pyridin im Schritt A) und/oder B) bei der Temperatur von 60°C und Konzentration der Pyridin-Lösung 60% (Vol.-%) durchgeführt wird.

4. Verfahren zur Herstellung nach dem Anspruch 2, **dadurch gekennzeichnet, dass** die Michael-Addition im Schritt D in n-Butanol bei 105°C, insbesondere über Nacht, durchgeführt wird.

5. Verfahren zur Herstellung nach dem Anspruch 2, **gekennzeichnet dadurch, dass** die Esterifikation in den Schritten B und/oder C insbesondere im Dichlormethan durchgeführt wird.

6. Verfahren zur Herstellung nach dem Anspruch 2, **dadurch gekennzeichnet, dass** die Schutzgruppen von Nukleosid, ausgewählt aus 2',3'-Bis(Dimethoxytrityl), 2',3'-Dibenzoyl und 2',3'-Diacetyl, im Schritt D durch eine ethanolische Methylamin-Lösung oder wässriges Ammoniak beseitigt werden.

7. Verfahren zur Herstellung nach dem Anspruch 2, **gekennzeichnet dadurch, dass** die 2',3'-Isopropyliden-Schutzgruppe im Schritt D durch das Einwirken von 0,5 mol.l⁻¹ Chlorwasserstoff im Methanol bei Raumtemperatur über Nacht beseitigt wird.

8. Lipophosphonoxine der allgemeinen Formel I oder deren Diastereomere oder pharmazeutisch annehmbare Salze und Hydrate, und/oder Gemische derartiger Verbindungen nach dem Anspruch 1 zur Verwendung als Arzneimittel.

9. Lipophosphonoxine der allgemeinen Formel I oder deren Diastereomere oder pharmazeutisch annehmbare Salze und Hydrate, und/oder Gemische derartiger Verbindungen nach dem Anspruch 1 zur Verwendung als antibakterielles Arzneimittel.

10. Lipophosphonoxine der allgemeinen Formel I oder deren Diastereomere oder pharmazeutisch annehmbare Salze und Hydrate, und/oder Gemische derartiger Verbindungen nach dem Anspruch 1 zur Verwendung als Wirkkomponenten von Desinfektionsmitteln und/oder selektiven Kultivierungsmedien für in vitro-Kultivierungen.

11. Antibakterielles Arzneimittel, Desinfektionsmittel und/oder selektives Kultivierungsmedium, enthaltend als Wirkkomponente mindestens ein Lipophosphonoxin der allgemeinen Formel I oder dessen Diastereomere oder pharamazeutische annehmbare Salze und Hydrate, und/oder Gemische derartiger Verbindungen nach dem Anspruch 1.

## Revendications

1. Lipophosphonoxines selon la formule générale I, 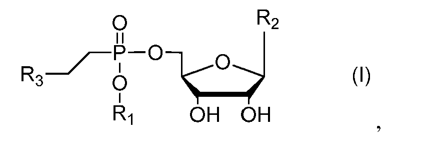 dans laquelle R₁ est sélectionné parmi un groupe comprenant (C₈-C₂₂) alkyle, hexadécyl-oxypropyle, tétradécyl-oxypropyle, tétradécyl-oxyéthyle, hexadécyl-oxyéthyle,
R₂ est sélectionné parmi un groupe comprenant l'uracile, thymine, cytosine,
R₃ est sélectionné parmi un groupe comprenant des composés de formules générales II et III : 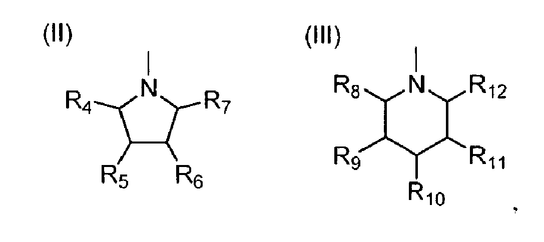 dans lesquelles R₄ représente H ou CH₂OH, R₅ représente H ou OH, R₆ représente H ou OH, R₇ représente H ou CH₂OH, R₈ représente H ou CH₂OH, R₉ représente H ou OH, R₁₀ représente H ou OH, R₁₁ représente H ou OH, R₁₂ représente H ou CH₂OH,
leurs diastéréomères et mélanges de leurs diastéréomères et leurs sels et hydrates pharmaceutiquement acceptables.

2. Procédé de préparation de lipophosphonoxines de formule générale I selon la revendication 1 ou de leurs diastéréomères et des mélanges de leurs diastéréomères, **caractérisé en ce qu'**il comprend les étapes A-D au cours desquelles
A) le diméthyl vinylphosphonate se réchauffe pendant la nuit avec la pyridine aqueuse de 40% à 70% (% de volume) à la température de 50 à 80 °C pour obtenir le méthyl vinylphosphanate **IX** 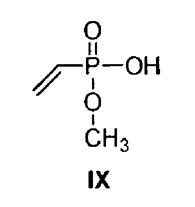
B) le méthyl vinylphosphanate obtenu estérifie avec le nucléoside **X,** protégé dans les positions 2'et 3' par un groupe protecteur, sélectionné parmi les 2',3'-isopropylidène, 2',3'-bis(diméthoxytrityle), 2',3'-dibenzoyle ou 2',3'-diacétyle, ayant un groupe 5'-hydroxyde libre 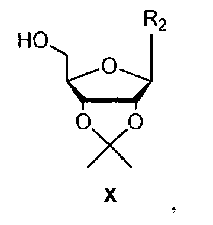 en présence du triisopropylbenzenesulfonyl chloride et sous catalyse par N-méthylimidazole dans un solvant, choisi parmi un groupe comprenant un acétonitrile, dioxane, tétrahydrofurane, dichloroéthane, chloroforme et dichlorométhane; l'ester méthylique obtenu est isolé par chromatographie sur un gel de silice en utilisant un gradient linéaire d'éthanol en chloroforme et consécutivement le groupe d'ester méthylique est éliminé par réchauffement pendant la nuit avec de la pyridine aqueuse de 40% à 70% (% de volume) à la température de 50 à 80 °C pour obtenir l'acide vinylphosphonique **XI** 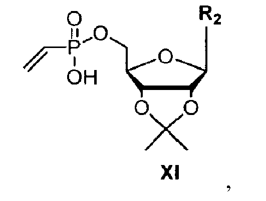 lequel est isolé par chromatographie sur un gel de silice en utilisant un gradient linéaire du mélange acétate d'éthyle : acétone : éthanol : eau en rapport des volumes 4:1:1:1, dans l'acétate d'éthyle ;
C) l'acide vinylphosphonique **XI** estérifie avec l'alcool à l'aide du triisopropylbenzensulfonyl chloride en tant qu'agent condensateur et sous catalyse par N-méthylimidazole dans un solvant, sélectionné parmi un groupe comprenant l'acétonitrile, le dioxane, le tétrahydrofurane, le dichloroéthane, le chloroforme et le dichlorométhane et le vinylphosphonate **XII** obtenu 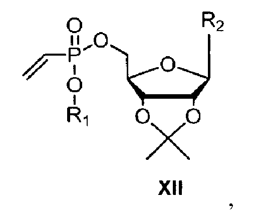 est séparé par chromatographie sur un gel de silice en utilisant un gradient d'éthanol en chloroforme ;
D) l'addition de Michael de l'amine secondaire sur la fonction vinyle du vinylphosphonate **XII** est effectuée par réchauffement dans un solvant organique sélectionné parmi un groupe comprenant l'acétonitrile, le dioxane, le tétrahydrofurane, le dichloroéthane, le chloroforme, l'éthanol, le propanol, l'isopropanol et le n-butanol à la température de 80 à 120 °C pendant une période de 4 à 12 heures, suivi de l'élimination du groupe protecteur sur le nucléoside; le produit est ensuite isolé par chromatographie sur un gel de silice en utilisant un gradient linéaire du mélange acétate d'éthyle: acétone: éthanol: eau en rapport des volumes 4:1:1:1 dans l'acétate d'éthyle; ou il est purifié à l'aide d'une chromatographie très efficace à la phase réversible.

3. Procédé de préparation selon la revendication 2, **caractérisé par** le réchauffement pendant la nuit avec la pyridine aqueuse à l'étape A) et/ou B) à la température de 60 °C et à la concentration de la solution de pyridine de 60 % (% de volume).

4. Procédé de préparation selon la revendication 2, **caractérisé en ce que** l'addition de Michael à l'étape D est effectuée dans le n-butanol à la température de 105 °C, de préférence pendant la nuit.

5. Procédé de préparation selon la revendication 2, **caractérisé en ce que** l'estérification aux étapes B et/ou C est de préférence effectuée dans le dichlorométhane.

6. Procédé de préparation selon la revendication 2, **caractérisé en ce que** les groupes protecteurs du nucléoside sélectionné parmi 2',3'-bis(diméthoxytrityle), 2',3'-dibenzoyle et 2',3'-diacétyle à l'étape D, sont éliminés par une solution éthanolique de méthylamine ou par l'ammoniaque aqueux.

7. Procédé de préparation selon la revendication 2, **caractérisé en ce que** le groupe protecteur 2',3'-isopropylidène à l'étape D est éliminé par un traitement avec 0.5 mol.l⁻¹ d'acide chlorhydrique dans le méthanol à la température ambiante du laboratoire pendant la nuit.

8. Lipophosphonoxines de formule générale I ou leurs diastéréomères ou leurs sels et hydrates pharmaceutiquement acceptables, et/ou mélanges de tels composés, selon la revendication 1, pour utilisation en tant que médicament.

9. Lipophosphonoxines de formule générale I ou leurs diastéréomères ou leurs sels et hydrates pharmaceutiquement acceptables, et/ou mélanges de tels composés, selon la revendication 1, pour utilisation en tant que médicament antibactérien.

10. Lipophosphonoxines de formule générale I ou leurs diastéréomères ou leurs sels et hydrates pharmaceutiquement acceptables, et/ou mélanges de tels composés, selon la revendication 1, pour utilisation en tant qu'agent actif des produits désinfectants et/ou des milieus sélectifs de croissance pour les cultures *in vitro.*

11. Médicament antibactérien, produit désinfectant et/ou milieu sélectif de croissance contenant en tant qu'agent actif au moins un lipophosphonoxine de formule générale I, ou ses diastéréomères et mélanges de ses diastéréomères ou ses sels et hydrates pharmaceutiquement acceptables, et/ou mélanges de tels composés, selon la revendication 1.
